# EUROPÄISCHE PATENTSCHRIFT

(11) **EP 2 480 574 B1**
(45) Veröffentlichungstag und Bekanntmachung des Hinweises auf die Patenterteilung: **13.08.2014**
(21) Anmeldenummer: 10762632.7
(22) Anmeldetag: 22.09.2010
(51) Int. Cl.: C07K 16/28

(54) **ANTI-CD33 ANTIKÖRPER UND IHRE ANWENDUNG ZUM IMMUNOTARGETING BEI DER BEHANDLUNG VON CD33-ASSOZIIERTEN ERKRANKUNGEN**
ANTI-CD33 ANTIBODIES AND THEIR USE FOR IMMUNOTARGETING IN THE TREATMENT OF CD33 ASSOSCIATED PATHOLOGIES
ANTICORPS ANTI-CD33 ET LEUR UTILISATION POUR IMMUNOTARGETING DANS LE TRAITEMENT DES PATHOLOGIES ASSOCIÉES A CD33

(30) Priorität: 25.09.2009 DE 102009045006
(43) Veröffentlichungstag der Anmeldung: 01.08.2012
(73) Patentinhaber: GEMoaB Monoclonals GmbH, 01307 Dresden (DE)
(72) Erfinder: BACHMANN, Michael, 65779 Kelkheim (DE); STAMOVA, Slava, 01277 Dresden (DE)
(74) Vertreter: Kailuweit & Uhlemann Patentanwälte
(86) Internationale Anmeldenummer: PCT/EP2010/063990
(87) Internationale Veröffentlichungsnummer: WO 2011/036183

(56) Entgegenhaltungen:
- EP-A1- 1 656 950
- WO-A2-2004/043344
- CN-A- 101 210 048
- US-A- 5 730 982
- WALTER ROLAND B ET AL: "Influence of CD33 expression levels and ITIM-dependent internalization on gemtuzumab ozogamicin-induced cytotoxicity.", BLOOD 1 FEB 2005 LNKD- PUBMED:15454492, Bd. 105, Nr. 3, 1. Februar 2005 (2005-02-01), Seiten 1295-1302, XP002612905, ISSN: 0006-4971
- LINENBERGER M L: "CD33-directed therapy with gemtuzumab ozogamicin in acute myeloid leukemia: progress in understanding cytotoxicity and potential mechanisms of drug resistance.", LEUKEMIA : OFFICIAL JOURNAL OF THE LEUKEMIA SOCIETY OF AMERICA, LEUKEMIA RESEARCH FUND, U.K FEB 2005 LNKD- PUBMED:15592433, Bd. 19, Nr. 2, Februar 2005 (2005-02), Seiten 176-182, XP002612906, ISSN: 0887-6924
- KOBAYASHI YUKIO ET AL: "Phase I/II study of humanized anti-CD33 antibody conjugated with calicheamicin, gemtuzumab ozogamicin, in relapsed or refractory acute myeloid leukemia: final results of Japanese multicenter cooperative study.", INTERNATIONAL JOURNAL OF HEMATOLOGY MAY 2009 LNKD- PUBMED:19360457, Bd. 89, Nr. 4, Mai 2009 (2009-05), Seiten 460-469, XP002612907, ISSN: 1865-3774
- LAMBA J K ET AL: "Coding polymorphisms in CD33 and response to gemtuzumab ozogamicin in pediatric patients with AML: a pilot study.", LEUKEMIA : OFFICIAL JOURNAL OF THE LEUKEMIA SOCIETY OF AMERICA, LEUKEMIA RESEARCH FUND, U.K FEB 2009 LNKD- PUBMED:18615103, Bd. 23, Nr. 2, Februar 2009 (2009-02), Seiten 402-404, XP002612908, ISSN: 1476-5551
- WELLHAUSEN S R ET AL: "CD33: BIOCHEMICAL AND BIOLOGICAL CHARACTERIZATION AND EVALUATION OF CLINICAL RELEVANCE", JOURNAL OF BIOLOGICAL REGULATORS AND HOMEOSTATIC AGENTS, WICHTIG EDITORE, MILAN, IT, Bd. 16, Nr. 2, 1. Januar 2002 (2002-01-01) , Seiten 139-143, XP008060453, ISSN: 0393-974X
- Lehmann Susann: "Bifunktionelle Proteine zum Reprogrammieren des Immunsystems", , 23. Januar 2008 (2008-01-23), XP002612909, Gefunden im Internet: URL:http://phpframe.wcms-file3.tu-dresden. de/generalize/index.php?g_nid=0111&node=21 1&e_id=9865&t_id=100 [gefunden am 2010-12-03]
- Walter, Roland Bruno: "Mechanism of endocytosis of CD33/Siglec-3: Role of ITIMs, tyrosine phosphorylation, and monoubiquitylation", Thesis, University of Washington, ISBN 9780542981357 , Mai 2007 (2007-05), XP002612910, Gefunden im Internet: URL:http://proquest.umi.com/pqdlink?did=12 46521681&Fmt=6&clientId=70702&RQT=309&VNam e=PQD [gefunden am 2010-12-06]
- RUDIKOFF S ET AL: "Single amino acid substitution altering antigen-binding specificity", PROCEEDINGS OF THE NATIONAL ACADEMY OF SCIENCES OF THE UNITED STATES (PNAS), NATIONAL ACADEMY OF SCIENCE, US, Bd. 79, 1. März 1982 (1982-03-01), Seiten 1979-1983, XP007901436, ISSN: 0027-8424, DOI: DOI:10.1073/PNAS.79.6.1979

### SEQUENCE LISTING

<110> Technische Universität Dresden
<120> Anti-CD33 Antikörper und ihre Anwendung zum Immunotargeting bei der Behandlung von CD33-assoziierten Erkrankungen
<130> 00017P0138DEWO
<160> 54
<170> PatentIn version 3.5
<210> 1
   <211> 5
   <212> PRT
   <213> Mus musculus
<400> 1
   Asp Tyr Val Val His
   1 5
<210> 2
   <211> 17
   <212> PRT
   <213> Mus musculus
<400> 2
   Tyr Ile Asn Pro Tyr Asn Asp Gly Thr Lys Tyr Asn Glu Lys Phe Lys
   1 5 10 15
   Gly
<210> 3
   <211> 11
   <212> PRT
   <213> Mus musculus
<400> 3
   Asp Tyr Arg Tyr Glu Val Tyr Gly Met Asp Tyr
   1 5 10
<210> 4
   <211> 10
   <212> PRT
   <213> Mus musculus
<400> 4
   Thr Ala Ser Ser Ser Val Asn Tyr Ile His
   1 5 10
<210> 5
   <211> 6
   <212> PRT
   <213> Mus musculus
<400> 5
   Thr Ser Lys Val Ala Ser
   1 5
<210> 6
   <211> 9
   <212> PRT
   <213> Mus musculus
<400> 6
   Gln Gln Trp Arg Ser Tyr Pro Leu Thr
   1 5
<210> 7
   <211> 5
   <212> PRT
   <213> Mus musculus
<400> 7
   Asp Tyr Val Leu His
   1 5
<210> 8
   <211> 17
   <212> PRT
   <213> Mus musculus
<400> 8
   Leu Ile Asn Thr Tyr Asn Gly Asp Val Arg Tyr Asn Gln Lys Phe Met
   1 5 10 15
   Gly
<210> 9
   <211> 11
   <212> PRT
   <213> Mus musculus
<400> 9
   Asp Tyr Arg Tyr Glu Tyr Tyr Ala Met Asp Tyr
   1 5 10
<210> 10
   <211> 10
   <212> PRT
   <213> Mus musculus
<400> 10
   Ser Ala Asn Ser Ser Val Ser Tyr Ile His
   1 5 10
<210> 11
   <211> 6
   <212> PRT
   <213> Mus musculus
<400> 11
   Thr Ser Lys Leu Ala Ser
   1 5
<210> 12
   <211> 9
   <212> PRT
   <213> Mus musculus
<400> 12
   Gln Gln Trp Thr Ser His Pro Leu Thr
   1 5
<210> 13
   <211> 120
   <212> PRT
   <213> Mus musculus
<400> 13
   Glu Val Lys Leu Gln Glu Ser Gly Pro Glu Leu Val Lys Pro Gly Ala
   1 5 10 15
   Ser Val Lys Met Ser Cys Lys Ala Ser Gly Tyr Lys Phe Thr Asp Tyr
   20 25 30
   Val Val His Trp Leu Lys Gln Lys Pro Gly Gln Gly Leu Glu Trp Ile
   35 40 45
   Gly Tyr Ile Asn Pro Tyr Asn Asp Gly Thr Lys Tyr Asn Glu Lys Phe
   50 55 60
   Lys Gly Lys Ala Thr Leu Thr Ser Asp Lys Ser Ser Ser Thr Ala Tyr
   65 70 75 80
   Met Glu Val Ser Ser Leu Thr Ser Glu Asp Ser Ala Val Tyr Tyr Cys
   85 90 95
   Ala Arg Asp Tyr Arg Tyr Glu Val Tyr Gly Met Asp Tyr Trp Gly Gln
   100 105 110
   Gly Thr Ser Val Thr Val Ser Ser
   115 120
<210> 14
   <211> 115
   <212> PRT
   <213> Mus musculus
<400> 14
   Asp Ile Val Leu Thr Gln Ser Pro Thr Ile Met Ser Ala Ser Pro Gly
   1               5                   10                  15
   Glu Arg Val Thr Met Thr Cys Thr Ala Ser Ser Ser Val Asn Tyr Ile
               20                  25                  30
   His Trp Tyr Gln Gln Lys Ser Gly Asp Ser Pro Lys Arg Trp Ile Phe
           35                  40                  45
   Asp Thr Ser Lys Val Ala Ser Gly Val Pro Ala Arg Phe Ser Gly Ser
       50                  55                  60
   Gly Ser Gly Thr Ser Tyr Ser Leu Thr Ile Ser Thr Met Glu Ala Glu
   65                  70                  75                  80
   Asp Ala Ala Thr Tyr Tyr Cys Gln Gln Trp Arg Ser Tyr Pro Leu Thr
                   85                  90                  95
   Phe Gly Asp Gly Thr Arg Leu Glu Leu Lys Arg Ala Asp Ala Ala Pro
               100                 105                 110
   Thr Val Ser
           115
<210> 15
   <211> 120
   <212> PRT
   <213> Mus musculus
<400> 15
   Glu Val Lys Leu Gln Glu Ser Gly Ala Glu Leu Val Arg Pro Gly Val
   1               5                   10                  15
   Ser Val Lys Ile Ser Cys Lys Gly Ser Gly Tyr Thr Phe Thr Asp Tyr
               20                  25                  30
   Val Leu His Trp Val Lys Gln Ser His Ala Lys Ser Leu Glu Trp Ile
           35                  40                  45
   Gly Leu Ile Asn Thr Tyr Asn Gly Asp Val Arg Tyr Asn Gln Lys Phe
       50                  55                  60
   Met Gly Lys Ala Thr Met Thr Ile Glu Lys Ser Ser Ser Thr Ala Tyr
   65                  70                  75                  80
   Met Glu Leu Val Arg Leu Thr Ser Glu Asp Ser Ala Ile Tyr Tyr Cys
                   85                  90                  95
   Ala Arg Asp Tyr Arg Tyr Glu Tyr Tyr Ala Met Asp Tyr Trp Gly Gln
               100                 105                 110
   Gly Thr Ser Val Thr Val Ser Ser
           115                 120
<210> 16
   <211> 115
   <212> PRT
   <213> Mus musculus
<400> 16
   Asp Ile Val Leu Thr Gln Ser Pro Ala Ile Met Ser Ala Ser Pro Gly
   1               5                   10                  15
   Glu Lys Val Thr Met Thr Cys Ser Ala Asn Ser Ser Val Ser Tyr Ile
               20                  25                  30
   His Trp Tyr Gln Gln Lys Ser Gly Thr Ser Pro Lys Arg Trp Ile Phe
           35                  40                  45
   Asp Thr Ser Lys Leu Ala Ser Gly Val Pro Ala Arg Phe Ser Gly Ser
       50                  55                  60
   Gly Ser Gly Thr Ser Tyr Ser Leu Thr Ile Ser Thr Met Glu Ala Glu
   65                  70                  75                  80
   Asp Ala Ala Thr Tyr Tyr Cys Gln Gln Trp Thr Ser His Pro Leu Thr
                   85                  90                  95
   Phe Gly Thr Gly Thr Lys Leu Gln Leu Lys Arg Ala Asp Ala Ala Pro
               100                 105                 110
   Thr Val Ser
           115
<210> 17
   <211> 15
   <212> DNA
   <213> Mus musculus
<400> 17
   gactatgttg tgcac 15
<210> 18
   <211> 51
   <212> DNA
   <213> Mus musculus
<400> 18
   tatattaatc cttacaatga tggtactaag tacaatgaga agttcaaagg c 51
<210> 19
   <211> 33
   <212> DNA
   <213> Mus musculus
<400> 19
   gactataggt acgaggtcta tggtatggac tac 33
<210> 20
   <211> 30
   <212> DNA
   <213> Mus musculus
<400> 20
   actgccagct caagtgtaaa ttacatacac 30
<210> 21
   <211> 21
   <212> DNA
   <213> Mus musculus
<400> 21
   gacacatcca aagtggcttc t 21
<210> 22
   <211> 27
   <212> DNA
   <213> Mus musculus
<400> 22
   cagcagtggc gaagttaccc tctcacg 27
<210> 23
   <211> 15
   <212> DNA
   <213> Mus musculus
<400> 23
   gattatgttt tacac 15
<210> 24
   <211> 51
   <212> DNA
   <213> Mus musculus
<400> 24
   cttattaata cttataatgg tgacgttagg tacaaccaga agttcatggg c 51
<210> 25
   <211> 33
   <212> DNA
   <213> Mus musculus
<400> 25
   gactataggt acgaatacta tgctatggac tac 33
<210> 26
   <211> 30
   <212> DNA
   <213> Mus musculus
<400> 26
   agtgccaact caagtgtcag ttacatacac 30
<210> 27
   <211> 18
   <212> DNA
   <213> Mus musculus
<400> 27
   acatccaaac tggcttct 18
<210> 28
   <211> 27
   <212> DNA
   <213> Mus musculus
<400> 28
   cagcagtgga ctagtcaccc actcacg 27
<210> 29
   <211> 360
   <212> DNA
   <213> Mus musculus
<400> 29
   gaggtcaagc tgcaggagtc aggacctgag ctggtaaagc ctggggcttc agtgaagatg     60
   tcctgcaagg cttctggata caaattcact gactatgttg tgcactggct gaagcagaag    120
   cctgggcagg gccttgagtg gattggatat attaatcctt acaatgatgg tactaagtac    180
   aatgagaagt tcaaaggcaa ggccacactg acttcagaca aatcctccag cacagcctac    240
   atggaggtca gcagcctgac ctctgaggac tctgcggtct attattgtgc aagagactat    300
   aggtacgagg tctatggtat ggactactgg ggtcaaggaa cctcagtcac cgtctcctca    360
<210> 30
   <211> 345
   <212> DNA
   <213> Mus musculus
<400> 30
   gacattgtgc tgacccagtc tccaacaatc atgtctgcat ctccagggga gagggtcacc     60
   atgacctgca ctgccagctc aagtgtaaat tacatacact ggtaccagca gaagtcaggc    120
   gactccccca aaagatggat tttcgacaca tccaaagtgg cttctggagt ccctgctcgc   180
   ttcagtggca gtgggtcagg gacctcttac tctctcacaa tcagtaccat ggaggctgaa    240
   gatgctgcca cttattactg ccagcagtgg cgaagttacc ctctcacgtt cggtgatggg    300
   accaggctgg agctgaaacg ggctgatgct gcaccaactg tatcc                    345
<210> 31
   <211> 360
   <212> DNA
   <213> Mus musculus
<400> 31
   gaggtcaagc tgcaggagtc aggggctgag ctggtgaggc ctggggtctc agtgaagatc        60
   tcctgcaagg gttctggcta cacattcact gattatgttt tacactgggt gaagcagagt       120
   catgcaaaga gtctagagtg gattggactt attaatactt ataatggtga cgttaggtac       180
   aaccagaagt tcatgggcaa ggccacaatg accatagaga aatcctccag cacagcctat       240
   atggaacttg tcagactgac atctgaggat tctgccatct attactgtgc aagagactat       300
   aggtacgaat actatgctat ggactactgg ggtcaaggaa cttcagtcac cgtctcctca       360
<210> 32
   <211> 345
   <212> DNA
   <213> Mus musculus
<400> 32
   gacattgtgc tgacccagtc tccagcaatc atgtctgcat ctccagggga gaaggtcacc        60
   atgacctgca gtgccaactc aagtgtcagt tacatacact ggtaccagca gaagtcaggc       120
   acttccccca aaagatggat ttttgacaca tccaaactgg cttctggagt ccctgctcgc       180
   ttcagtggca gtgggtctgg gacctcttac tctctcacaa tcagcaccat ggaggctgaa       240
   gatgctgcca cttattactg ccagcagtgg actagtcacc cactcacgtt cggtactggg       300
   accaagctgc agctgaaacg ggctgatgct gcaccaactg tatcc                       345
<210> 33
   <211> 18
   <212> PRT
   <213> Mus musculus
<400> 33
   Gly Leu Ile Asn Thr Tyr Asn Gly Asp Val Arg Tyr Asn Gln Lys Phe
   1               5                   10                  15
   Met Gly
<210> 34
   <211> 18
   <212> PRT
   <213> Mus musculus
<400> 34
   Leu Ile Asn Thr Tyr Asn Gly Asp Val Arg Tyr Asn Gln Lys Phe Met
   1               5                   10                  15
   Gly Lys
<210> 35
   <211> 19
   <212> PRT
   <213> Mus musculus
<400> 35
   Gly Leu Ile Asn Thr Tyr Asn Gly Asp Val Arg Tyr Asn Gln Lys Phe
   1 5 10 15
   Met Gly Lys
<210> 36
   <211> 7
   <212> PRT
   <213> Mus musculus
<400> 36
   Asp Thr Ser Lys Leu Ala Ser
   1 5
<210> 37
   <211> 5
   <212> PRT
   <213> Mus musculus
<400> 37
   Ser Tyr Tyr Ile His
   1 5
<210> 38
   <211> 17
   <212> PRT
   <213> Mus musculus
<220>
   <221> misc_feature
   <222> (16)..(16)
   <223> Xaa can be any naturally occurring amino acid
<400> 38
   Val Ile Tyr Pro Gly Asn Asp Asp Ile Ser Tyr Asn Gln Lys Phe Xaa
   1 5 10 15
   Gly
<210> 39
   <211> 9
   <212> PRT
   <213> Mus musculus
<400> 39
   Glu Val Arg Leu Arg Tyr Phe Asp Val
   1 5
<210> 40
   <211> 17
   <212> PRT
   <213> Mus musculus
<400> 40
   Lys Ser Ser Gln Ser Val Phe Phe Ser Ser Ser Gln Lys Asn Tyr Leu
   1 5 10 15
   Ala
<210> 41
   <211> 7
   <212> PRT
   <213> Mus musculus
<400> 41
   Trp Ala Ser Thr Arg Glu Ser
   1 5
<210> 42
   <211> 8
   <212> PRT
   <213> Mus musculus
<400> 42
   His Gln Tyr Leu Ser Ser Arg Thr
   1 5
<210> 43
   <211> 5
   <212> PRT
   <213> Mus musculus
<400> 43
   Asp Tyr Asn Met His
   1 5
<210> 44
   <211> 17
   <212> PRT
   <213> Mus musculus
<400> 44
   Tyr Ile Tyr Pro Tyr Asn Gly Gly Thr Gly Tyr Asn Gln Lys Phe Lys
   1 5 10 15
   Ser
<210> 45
   <211> 7
   <212> PRT
   <213> Mus musculus
<400> 45
   Gly Arg Pro Ala Met Asp Tyr
   1               5
<210> 46
   <211> 15
   <212> PRT
   <213> Mus musculus
<400> 46
   Arg Ala Ser Glu Ser Val Asp Asn Tyr Gly Ile Ser Phe Met Asn
   1               5                   10                  15
<210> 47
   <211> 6
   <212> PRT
   <213> Mus musculus
<400> 47
   Ala Ser Asn Gln Gly Ser
   1               5
<210> 48
   <211> 9
   <212> PRT
   <213> Mus musculus
<400> 48
   Gln Gln Ser Lys Glu Val Pro Trp Thr
   1               5
<210> 49
   <211> 5
   <212> PRT
   <213> Mus musculus
<400> 49
   Asp Tyr Asn Met Tyr
   1               5
<210> 50
   <211> 17
   <212> PRT
   <213> Mus musculus
<400> 50
   Tyr Ile Asp Pro Tyr Lys Gly Gly Thr Ile Tyr Asn Gln Lys Phe Lys
   1 5 10 15
   Gly
<210> 51
   <211> 10
   <212> PRT
   <213> Mus musculus
<400> 51
   Gln Met Ile Thr Ala Tyr Tyr Phe Asp Tyr
   1 5 10
<210> 52
   <211> 11
   <212> PRT
   <213> Mus musculus
<400> 52
   Lys Ala Ser Gln Asp Ile Asn Lys Tyr Ile Ala
   1 5 10
<210> 53
   <211> 6
   <212> PRT
   <213> Mus musculus
<400> 53
   Thr Ser Thr Leu Gln Pro
   1 5
<210> 54
   <211> 8
   <212> PRT
   <213> Mus musculus
<400> 54
   Leu Gln Tyr Asp Asn Leu Leu Thr
   1 5

## Beschreibung

Die Erfindung betrifft Antikörper gegen das tumorassoziierte Antigen CD33 und ihre Anwendung zum Immunotargeting von CD33-positiven Zellen. Die erfindungsgemäßen Antikörper eignen sich zur Anwendung im Bereich der Medizin, der Pharmazie und in der biomedizinischen Forschung.

Krebs (maligne Neoplasie) ist eine Klasse von Erkrankungen, bei denen sich eine Gruppe von Zellen durch unkontrollierte Zellteilung, durch Invasion in und Zerstörung von anliegenden Geweben und manchmal durch Metastasierung auszeichnet. Diese malignen Eigenschaften von Krebs unterscheiden ihn von benignen Tumoren, welche durch ihr Wachstum umliegendes Gewebe zwar verdrängen, dieses aber nicht infiltrieren und welche nicht metastasieren. Die meisten Krebsarten bilden Tumoren, manche nicht, wie Leukämien, die das blutbildende System betreffen. Krebs im Sinne der Erfindung bezeichnet hier sowohl maligne Tumore als auch Hämoblastosen.

Bei Krebszellen ist die Abstimmung von Wachstum, Teilung und Zerstörung im Zellverband außer Kraft gesetzt. Sie entstehen, wenn sich bestimmte Gene verändern, diese Veränderungen nicht mehr repariert werden, was zur Unfunktionalität bestimmter Genbereiche führt, so dass sich die Krebszellen selber zur Teilung stimulieren und wachstumshemmende Signale aus der Zell-Umgebung ignorieren.

Grundsätzlich versucht das Immunsystem, die unkontrolliert wachsenden Zellen zu bekämpfen. Problematisch ist hierbei die Tatsache, dass Krebszellen den normalen Körperzellen in vieler Hinsicht gleichen, da sie aus solchen hervorgegangen sind. Daher sind die Abwehrmaßnahmen des Immunsystems meist nicht ausreichend, um das Wachstum des Tumors zu kontrollieren.

Bei der akuten myeloischen Leukämie (AML) ist die Myelopoese betroffen, also der Teil des blutbildenden Systems, der für die Bildung von Granulozyten und Monozyten verantwortlich ist. Die Myeloblasten stellen in der Hämatopoese eine unreife Vorstufe der myeloiden weißen Blutzellen dar. In AML resultierten mehrere genetische Veränderungen, die die für die Zellteilung relevanten Gene betreffen, in einem einzelnen Myeloblasten zur Bildung einer Zelle, die in einem unreifen Zustand verbleibt und sich massiv vermehrt. Diese Vermehrung der unreifen Vorstufen im Knochenmark und auch im Blut kennzeichnen AML.

Die Veränderungen können an einer Reihe verschiedener Stellen des Zellzyklus erfolgen, so dass AML verschiedene phänotypische, genotypische und klinische Eigenschaften aufweist. Die moderne Klassifikation von AML basiert auf der These, dass die Eigenschaften und das Verhalten der Tumorzellen darauf ankommt, an welcher Stufe des Zellzyklus die Proliferation gestoppt wurde. In vielen AML-Patienten können bestimmte spezifische zytogenetische Auffälligkeiten festgestellt werden, welche oft von prognostischer Signifikanz sind. Die genetischen Veränderungen kodieren für abnorme Fusionsproteine, meist Transkriptionsfaktoren, welche die unkontrollierte Proliferation verursachen.

Tumorzellen unterscheiden sich von den gesunden Zellen durch die Expression von Tumorantigenen, also von Proteinen, die nur die Tumorzellen exprimieren. Diese entstehen als Folge des in Krebszellen veränderten Genoms, beziehungsweise durch eine veränderte Genexpression. Tumorantigene befinden sich an der äußeren Zellmembran der Tumorzellen, im Zellplasma und im Zellkern. Die Tumorantigene sind als Zielstruktur die Basis für die meisten Konzepte der Krebsimmuntherapie. Bisher sind über 2000 Tumorantigene bekannt.

Ideal als Target für die Krebstherapie sind tumorspezifische Antigene (TSA), also Antigene, die nur von Krebszellen und nicht von gesunden Zellen produziert werden. Die meisten Tumorantigene sind jedoch nicht tumorspezifisch, sondern tumorassoziiert (TAA), d.h. dass sie auch von gesunden Zellen exprimiert werden. Allerdings sind bei vielen Tumoren die Tumorantigene überexprimiert. Durch Mutationen im Genom können auch strukturelle Veränderungen in der Proteinsequenz auftreten. Viele Tumorantigene treten nur bei bestimmten Tumorarten und dort oft auch nur in bestimmten Fällen auf.

Bei AML vorhandene Tumorantigene umfassen unter anderem TAA, die mit einer Reihe weiterer Tumorerkrankungen assoziiert sind, wie z.B. Telomerase Reverse Transkriptase, Wilms's Tumor 1 (WT1)-Protein und Survivin. Außerdem sind auch TAA bekannt, die mit anderen leukämischen Erkrankungen assoziiert sind, wie CD168 und M-Phase-Phosphoprotein. Weiterhin gibt es TAA, die besonders im Zusammenhang mit AML auftreten, wie CD33, CD45 und mHAgs (minor histocompatibility antigens).

CD33 ist ein glykosyliertes Transmembranprotein aus 364 Aminosäuren, welches zur Familie der sialinsäurebindenden Ig-verwandten Lektine (SIGLECs) gehört. Es wird auch frühen hämatopoetischen Vorläuferzellen, myelomonozytischen Vorläufern und myeloiden Zellen exprimiert, wohingegen es nicht auf normalen pluripotenten Knochenmarkstammzellen vorhanden ist. Etwa 85-90% der AML-Patienten sind positiv für CD33. Daher stellt CD33 ein besonders attraktives Target für eine Immuntherapie dar und wird bereits in einer gezielten Immuntherapie als Target verwendet.

Die konventionelle AML-Therapie ist eine Chemotherapie zur Induktion einer Remission, danach können weitere Chemotherapien oder die Transplantation hämatopoetischer Stammzellen folgen. In den Vereinigten Staaten wurde im Jahr 2000 ein monoklonaler Antikörper konjugiert mit einem Zytostatikum, Gemtuzumab Ozogamicin, für Patienten mit rezidiver AML zugelassen, die keine Kandidaten für die Standard-Chemotherapie sind.

Antikörper sind Proteine aus der Klasse der Globuline, die in Wirbeltieren als Reaktion auf bestimmte Stoffe, die Antigene, gebildet werden. Bei der Bekämpfung von Krebszellen spielen die körpereigenen polyklonalen Antikörper nur eine sehr geringe Rolle. Dies resultiert daraus, dass die meisten Tumorzellen nicht ausreichend stark veränderte Antigene präsentieren, die durch das eigene Immunsystem als fremd erkannt werden, so dass körpereigene Antikörper nicht in ausreichender Zahl an Tumorzellen binden. Monoklonale Antikörper sind in ihrer Struktur völlig identisch und nur gegen ein Epitop eines Antigens ausgerichtet.

Zur Krebstherapie eingesetzte monoklonale Antikörper wirken fast ausschließlich über die antikörperabhängige zellvermittelte Zytotoxizität (antibody dependent cellular cytotoxicity, ADCC). Dabei rekrutieren Antikörper über ihren Fc-Teil zytotoxische Effektorzellen, wie beispielsweise NK-Zellen, Makrophagen, Lymphozyten oder Granulozyten, die selbst keine Antigenspezifität aufweisen, zur Tumorzelle. Die direkte Schädigung der Zelle durch angebundene monoklonale Antikörper, wie die Auslösung bestimmter intrazellulärer Signalkaskaden die die Apoptose einleiten durch die Kreuzvernetzung der Tumorantigene aufgrund der Antikörperbindung, ist die seltene Ausnahme.

Um die Wirkung von monoklonalen Antikörpern bei Krebserkrankungen zu verstärken, wurden verschiedene Strategien des Drug Targetings entwickelt, um die Antikörper als Träger für potentere Wirkstoffe zu nutzen, sogenannte Immunkonjugate oder Antikörperderivate. Dafür wurden Wirkstoffe, wie Radionuklide, Toxine (beispielsweise das Diphtherietoxin), Zytokine oder auch Zytostatika an die entsprechenden Antikörper gebunden (konjugiert).

Dabei ist es prinzipiell möglich, an Antikörper hochpotente Wirkstoffe zu binden, die bei freier systemischer Gabe aufgrund ihrer hohen Toxizität zu nicht vertretbaren Nebenwirkungen führen würden. Die Antikörperderivate bestehen aus drei Komponenten: einem Antikörper bzw. Antikörperfragment, einem Wirkstoff und einem Verbindungsstück (Linker) zwischen Antikörper und Wirkstoff.

WO 2004/043344 A2 offenbart den anti-CD33 Antikörper "My9-6" und Verfahren zur Behandlung von akuter myeloischer Leukämie (AML) unter Einsatz dieses Antikörpers. Es ist offenbart, dass der anti-CD33 zum therapeutischen Einsatz bevorzugt in veränderter Form vorliegt. So sind humanisierte Antikörperderivate oder allein die epitopbindenden Fragmente des Antikörpers My9-6 offenbart. Weiter ist offenbart, dass der My9-6 Antikörper mit einer therapeutisch wirksamen Substanz konjugiert sein kann.

Der erste klinisch untersuchte gegen CD33 gerichtete Antikörper ist M195 (US 5,730,982, EP1656950 A1). Die Humanisierte Variante dieses Antikörpers des Isotypen IgG1, auch unter dem Namen Lintuzumab bekannt, wurde in klinischen Studien getestet, wobei in Phase III jedoch kein Vorteil von Lintuzumab in Kombination mit Chemotherapie im Vergleich zu alleiniger Chemotherapie festgestellt werden konnte (E. J. Feldman et. al., Phase III Randomized Multicenter Study of a Humanized Anti-CD33 Monoclonal Antibody, Lintuzumab, in Combination With Chemotherapy, Versus Chemotherapy Alone in Patients With Refractory or First-Relapsed Acute Myeloid Leukemia. J. Clin. Oncol. 23 (18), 2005, S. 4110-4116).

Gemtuzumab-Ozogamicin ist ein Immunkonjugat eines gegen CD33 gerichteten monoklonalen Antikörpers vom Typ IgG4, das bis 2010 in den USA zur Behandlung von AML eingesetzt wurde, mittlerweile jedoch vom Markt genommen wurde. Es besteht aus einem humanisierten monoklonalen Antikörper gegen CD33, welcher mit einem Zytostatikum, Calicheamicin, konjugiert ist. Der Wirkmechanismus beruht auf der Endozytose des Antikörperderivats, so dass das daran gekoppelte Zytostatikum nach der Aufnahme in die Zelle seine toxische Wirkung entfalten kann (Walter R et al. Blood. 105 (3), 2005, S. 1295-1302). In Europa wurde Gemtuzumab-Ozogamicin nicht zugelassen, da die Wirksamkeit nach Ansicht der Europäischen Zulassungsbehörde EMEA nicht ausreichend belegt ist.

Die folgende Tabelle zeigt die Aminosäuresequenzen der CDRs (complementarity determining regions, die Antigenbindungsstellen eines Antikörpers) der aus den entsprechenden Dokumenten bekannten anti-CD33-Antikörper:

| Dokument | V_{H} CDR1 | SEQ ID Nr. | V_{H} CDR2 | SEQ ID Nr. | V_{H} CDR3 | SEQ ID Nr. |
|---|---|---|---|---|---|---|
| US 7,557,189 | SYYIH | 37 | VIYPGNDDISYNQKFXG | 38 | EVRLRYFDV | 39 |
| US 5,730,982 | DYNMH | 43 | YIYPYNGGTGYNQKFKS | 44 | GRPAMDY | 45 |
| CN101210048 | DYNMY | 49 | YIDPYKGGTIYNQKFKG | 50 | QMITAYYFDY | 51 |
| | | | | | | |

| | V_{L} CDR1 | | V_{L} CDR2 | | V_{L} CDR3 | |
|---|---|---|---|---|---|---|
| US 7,557,189 | KSSQSVFFSSSQKNYLA | 40 | WASTRES | 41 | HQYLSSRT | 42 |
| US 5,730,982 | RASESVDNYGISFMN | 46 | ASNQGS | 47 | QQSKEVPWT | 48 |
| CN101210048 | KASQDINKYIA | 52 | TSTLQP | 53 | LQYDNLLT | 54 |

Eine generelle Problematik für die therapeutische Wirksamkeit der monoklonalen Antikörper ist das Bindungsvermögen der Antikörper an die Krebszellen, also die Affinität der Antikörper. Auch bei Krebszellen, die ausreichend Tumorantigene präsentieren, ist die Bindungsrate oft nicht ausreichend hoch. Mit einer Molekülmasse von etwa 150 kDa sind Antikörper außerdem generell in ihrer Gewebegängigkeit eingeschränkt. In diesem Fall haben Antikörperfragmente, wie Fab, F(ab)₂ oder scFv (single chain variable fragments), aufgrund ihrer deutlich geringeren Größe erhebliche Vorteile.

Bispezifische Antikörper, also Antikörperderivate aus Bestandteilen zweier unterschiedlicher monoklonaler Antikörper, bieten neue Möglichkeiten für Therapiekonzepte in der Krebsimmuntherapie.

Quadroma sind bispezifische Antikörper der ersten Generation und bestehen aus je einer schweren und einer leichten Kette zweier unterschiedlicher monoklonaler Antikörper. Die beiden Arme des Antikörpers sind dabei gegen jeweils andere Antigene gerichtet. Der Fc-Teil wird gemeinsam aus den beiden schweren Ketten der Antikörper gebildet. Durch diesen Aufbau ist es beispielsweise möglich das Paratop eines gegen ein Tumorantigen gerichteten Antikörpers und das Paratop eines anderen gegen ein Lymphozyten-Antigen gerichteten Antikörpers, auf je einem Arm des bispezifischen Antikörpers zu platzieren. Es ist so möglich, einen Drei-Zell-Komplex zu bilden, der aus den jeweils durch die unterschiedlichen Paratope gebundenen Zellen und der an den Fc-Teil gebundenen Effektorzelle ergibt. Dabei ergibt sich im Regelfall eine verbesserte Aktivierung der körpereigenen Immunzellen gegenüber den Tumorzellen.

Bispezifische Antikörper der neueren Generation sind aus zwei unterschiedlichen scFv-Fragmenten aufgebaut. Diese sind untereinander mit einem Linker verbunden. So kann ein bispezifischer Antikörper beispielsweise mit einem scFv an Tumorzellen und mit dem anderen scFv an Effektorzellen binden.

Wird ein Paratop gegen T-Zellen gerichtet, so können auch diese Zellen aktiviert werden. Mit normalen monoklonalen Antikörpern ist dies nicht möglich, da T-Zellen über keine Fc-Rezeptoren verfügen. Bispezifische Antikörper weisen darüber hinaus ein höheres zytotoxisches Potenzial auf. Sie binden auch an Antigene, die relativ schwach exprimiert werden.

Bis heute sind noch keine bispezifischen Antikörper für die klinische Anwendung beim Menschen zugelassen.

Es sind bispezifische Antikörper bekannt, bei denen ein scFv an den CD3-Komplex auf T-Zellen bindet, diese werden auch als BiTE bezeichnet (bispecific T-cell engager) (P. A. Baeuerle et. al., BiTE: Teaching antibodies to engage T cells for cancer therapy. Curr Opin Mol Ther 11, 2009, S. 22-30).

Momentan befinden sich zwei unterschiedliche BiTE-Antikörper in klinischen Studien. Blinatumomab, ein Antikörper der gegen CD3 und CD19 gerichtet ist, wird bei Patienten in späten Phasen des Non-Hodgkin-Lymphoms und bei Patienten mit akuter lymphoblastischer Leukämie der B-Zellreihe (B-ALL) getestet. MT110 ist ein Antikörper, der gegen CD3 und EpCAM (epithelial cell adhesion molecule) gerichtet ist und wird bei Patienten mit Bronchialkarzinom und Patienten mit gastrointestinalen Krebserkrankungen getestet (R. Bargou en. al., Tumor regression in cancer patients by very low doses of a T cell-engaging antibody. Science 321, 2008, S. 974-977; K. Brischwein et.al., MT110: a novel bispecific single-chain antibody construct with high efficacy in eradicating established tumors. Mol Immunol 43, 2006, S. 1129-1143).

Nicht alle monoklonalen Antikörper eignen sich in der Form von scFv-Fragmenten oder für die Konstruktion von bispezifischen Konstrukten. Hier ist besonders die Affinität der Antikörper entscheidend, die durch die variablen Regionen bestimmt wird. Nur besonders hochaffine Antikörper eignen sich als scFv-Fragmente, da die Bindung an das jeweilige Antigen nur mit einem Paar aus variablen Regionen der schweren und leichten Ketten erfolgt, im Gegensatz zu kompletten IgG-Antikörpern, die zwei Paare aus variablen Regionen der schweren und leichten Ketten besitzen.

Für die Behandlung von AML und anderen Karzinomen besteht Bedarf an neuen therapeutischen Konzepten. Bisherige bekannte Antikörper besitzen eine zu geringe Affinität zu tumorassoziierten Antigenen, wie CD33, um therapeutisch eingesetzt werden zu können. Daher besteht Bedarf an der Entwicklung von spezifischen Antikörpern für tumorassoziierte Antigene, z.B. CD33, die eine hohe Affinität besitzen und somit zur Nutzung in der Krebstherapie geeignet sind.

Aufgabe der Erfindung ist daher die Bereitstellung von neuen anti-CD33 Antikörpern, insbesondere mit einer hohen Affinität zu CD33, die es ermöglicht die Antikörper als rekombinante Fragmente zum Immunotargeting zu verwenden.

Erfindungsgemäß wird die Aufgabe gelöst durch neue anti-CD33 Antikörper, welche komplementaritätsbestimmende Regionen (complementary determining regions, CDRs) enthalten, die dadurch gekennzeichnet sind, dass die CDRs der variablen Region der schweren Kette (V_{H}) die folgenden Sequenzen umfassen:
- CDR1: DYVVH (SEQ ID Nr. 01),
- CDR2: YINPYNDGTKYNEKFKG (SEQ ID Nr. 02),
- CDR3: DYRYEVYGMDY (SEQ ID Nr. 03),
und dass die CDRs der variablen Region der leichten Kette (V_{L}) die folgenden Sequenzen umfassen
- CDR1: TASSSVNYIH (SEQ ID Nr. 04),
- CDR2: TSKVAS (SEQ ID Nr. 05),
- CDR3: QQWRSYPLT (SEQ ID Nr. 06).

Die erfindungsgemäßen anti-CD33 Antikörper, hierin auch bezeichnet als "anti-CD33DRB2", sind in ihren Aminosäuresequenzen der schweren und der leichten Ketten, ihren CDRs und in Methoden zur Expression des Antikörpers in rekombinanter Form charakterisiert.

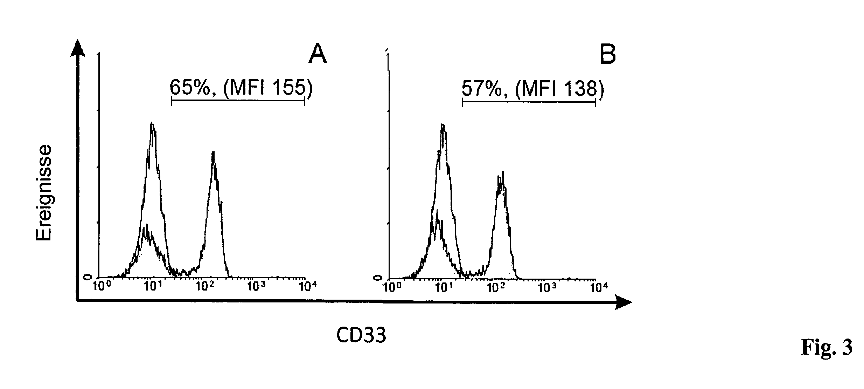

Weiterhin sind die anti-CD33DRB2 Antikörper funktionell charakterisiert und es wurde gezeigt, dass sie sich durch eine hohe Affinität für humanes CD33 auszeichnen (Figur 3). Die erfindungsgemäßen Antikörper weisen daher vorteilhaft eine hohe Affinität für humanes CD33 auf. Die Affinitäten der nativen Antikörper liegen in der Größenordnung von 10⁻¹⁰ mol/l. Die Affinitäten der rekombinanten Derivate erreichen 10⁻⁷ bis 10⁻⁸ mol/l. Es konnte gezeigt werden, dass die Antikörper zum überwiegenden Teil nicht durch Endozytose in die CD33-positiven Zellen aufgenommen werden und über einen langen Zeitraum an der Zelloberfläche verbleiben. Dies macht sie für einen therapeutischen Einsatz zum Immunotargeting von Effektorzellen an CD33-positive Zellen besonders geeignet. Durch die hohe Affinität eignen sich die erfindungsgemäßen CDR-Sequenzen insbesondere zur Herstellung von rekombinanten Fragmenten (wie scFv) und zum Immunotargeting.

Der Begriff "Antikörper" im erfindungsgemäßen Sinn umfasst alle Antikörper, Antikörperfragmente und Derivate derselben, die in der Lage sind, an das Antigen, in diesem Fall CD33, zu binden und die erfindungsgemäßen CDRs komplett aufweisen. Dazu zählen die kompletten monoklonalen Antikörper und auch die epitopbindenden Fragmente dieser Antikörper. Hierbei umfassen die epitopbindenden Fragmente (hier auch als Antikörperfragmente oder Antikörperderivate bezeichnet) alle Teile des Antikörpers, die in der Lage sind, an das Antigen, in diesem Fall CD33, zu binden. Beispiele für erfindungsgemäß bevorzugte Antikörperfragmente beinhalten, sind aber ausdrücklich nicht limitiert auf, Fab, Fab', F(ab')₂, Fd, Einzelketten (single-chain) variable Fragmente (scFv), Einzelketten-Antikörper, disulfidverlinkte variable Fragmente (sdFv) und Fragmente die entweder eine variable Region der leichten Kette (V_{L}) oder eine variable Region der schweren Kette (V_{H}) beinhalten. Weiterhin zählen dazu rekombinant hergestellte Antikörper, wie Diabodies, Triabodies und Tetrabodies.

Bevorzugt trägt der Antikörper ein Markermolekül, wie z. B. Biotin, Dioxygenin, ein Radionuklid oder einen Fluoreszenzfarbstoff. Besonders bevorzugt ist der Antikörper mit einer Effektorgruppe konjugiert.

Antikörperfragmente enthalten die variablen Regionen entweder allein oder in Kombination weiteren Regionen, die ausgewählt sind aus der Hinge-Region, und dem ersten, zweiten und dritten Bereich der konstanten Region (C_{H}1, C_{H}2, C_{H}3). Ebenfalls durch den Begriff "Antikörper" umfasst sind chimäre Antikörper, bei denen unterschiedliche Teile des Antikörpers aus verschiedenen Spezies stammen, wie beispielsweise Antikörper mit einer murinen variablen Region, welche mit einer humanen konstanten Region kombiniert ist.

Antikörperfragmente sind gegebenenfalls untereinander über einen Linker verknüpft. Der Linker umfasst eine kurze (bevorzugt 10 bis 20 Aminosäurereste lange), flexible Peptidsequenz, die so ausgewählt wird, dass das Antikörperfragment eine derartige dreidimensionale Faltung der V_{L} und V_{H} besitzt, dass es die Antigenspezifität des kompletten Antikörpers aufweist. Bevorzugte Linker, aber nicht exklusiv, sind Glycin-Serin-Linker mit der Struktur (GlyₓSer)_{y} mit x und y ausgewählt aus 1 bis 10, bevorzugt 3 bis 5. Weiterhin werden Linker bevorzugt, die aus einer Peptidsequenz bestehen, welche die Proteaseresistenz der Antikörperderivate erhöhen können.

Die Bezeichnung "variable Region" ist erfindungsgemäß definiert als die Teile der schweren und leichten Ketten der Antikörper, die sich in ihrer Sequenz zwischen Antikörpern unterscheiden und die Spezifität des Antikörpers und die Bindung an sein Antigen bestimmen. Die Variabilität ist hierbei nicht gleichmäßig in der variablen Region verteilt. Sie ist üblicherweise innerhalb dreier definierter Segmente der variablen Region konzentriert, die als komplementaritätsbestimmende Regionen (CDRs) oder auch hypervariable Regionen bezeichnet werden und in den variablen Regionen sowohl der leichten als auch der schweren Ketten vorhanden sind. Die stärker konservierten Teile der variablen Regionen werden Gerüstregionen (framework regions) genannt. Die variablen Regionen der schweren und leichten Ketten enthalten vier Gerüstregionen, die überwiegend eine beta-Faltblatt Struktur annehmen, wobei jede Gerüstregion mit drei CDRs verbunden ist, die Schleifen bilden, die die beta-Faltblatt-Strukturen verbinden und in manchen Fällen Teil der beta-Faltblatt-Struktur sind. Die CDRs der jeweiligen Kette sind durch die Gerüstregionen in unmittelbare Nähe gebracht und tragen gemeinsam mit den CDRs der anderen Kette zur Bildung der Antigenbindungsregion der Antikörper bei.

Die konstante Region (Fc) der Antikörper ist nicht an der Antigenbindung beteiligt, sondern bietet stattdessen vielfältige Effektorfunktionen, die durch die Bindung an die entsprechenden Fc-bindenden Rezeptoren ausgelöst werden, wie beispielsweise die Induktion der antikörperabhängige zellvermittelte Zytotoxizität (ADCC).

Bevorzugt umfassen die erfindungsgemäßen Antikörper mindestens eine variable Region der schweren Kette (V_{H}) und eine variable Region der leichten Kette (V_{L}) in Form eines scFv. Die variable Region der schweren Kette (V_{H}) und die variable Region der leichten Kette (V_{L}) enthalten dabei jeweils mindestens eine der erfindungsgemäßen CDR-Sequenzen.

Erfindungsgemäß bevorzugt werden Antikörper, die folgende Strukturen enthalten, die gegebenenfalls humanisiert sind:
- eine variable Region der schweren Kette gemäß der Sequenz SEQ ID Nr. 13 und eine variable Region der leichten Kette gemäß der Sequenz SEQ ID Nr. 14.

In den Antikörpern können bestimmte Aminosäuren der spezifischen Aminosäuresequenzen so ausgetauscht sein, dass sie die Bindungseigenschaften des CD33 Antikörpers beibehalten, sich jedoch in ihrer Sequenz durch Austausch, Deletion oder Addition einer oder mehrerer Aminosäuren unterscheiden. Beschrieben sind somit auch Antikörper, die Strukturen enthalten, deren Aminosäuresequenzen zu den erfindungsgemäßen Aminosäuresequenzen der variablen Regionen gemäß SEQ ID Nr. 13 und 14 eine Sequenzidentität von bevorzugt mindestens 70%, besonders bevorzugt mindestens 80%, insbesondere mindestens 90% aufweisen und die das Antigen CD33 binden, wobei diese Sequenzen mindestens eine der erfindungsgemäßen CDRs gemäß SEQ ID Nr. 1 bis 6 aufweisen.

In besonderen Ausgestaltungen der Erfindung umfasst der Antikörper folgende Strukturen:
- eine variable Region der schweren Kette mit der Sequenz gemäß SEQ ID Nr. 13 und eine variable Region der leichten Kette mit der Sequenz gemäß SEQ ID Nr. 14; und
- eine konstante Region einer schweren Kette eines humanen IgG,
- eine Region C_{L} der humanen leichten Kappa-Kette
- und eine humane IgG3 Hinge-Region.

Gegebenenfalls liegt der Antikörper in Form eines F(ab')2-Fragments vor.

In einer besonders bevorzugten Ausgestaltung der Erfindung werden Antikörper in Form von scFv-Fragmenten welche mindestens eine variable Region der schweren Kette (V_{H}) und eine variable Region der leichten Kette (V_{L}) umfassen, die die erfindungsgemäßen CDR-Regionen enthalten. Weiterhin besonders bevorzugt werden Antikörper in Form von scFv-Fragmenten welche mindestens eine der erfindungsgemäßen variablen Regionen der schweren und leichten Kette umfasst.

Die Erfindung umfasst murine anti-CD33 Antikörper und humanisierte Versionen dieser Antikörper.

Das Ziel der Humanisierung von Antikörpern liegt in der Reduktion der Immunogenität eines xenogenen Antikörpers, wie in diesem Fall des murinen Antikörpers, zur Verwendung im humanen System, wobei die volle Bindungsaffinität und die Antigenspezifität erhalten bleibt. Humanisierte Antikörper können auf verschiedenen Wegen hergestellt werden, wie beispielsweise durch Resurfacing und CDR-Grafting. Beim Resurfacing werden durch eine Kombination aus Molecular Modelling, statistischen Analysen und Mutagenese alle nicht-CDR-Regionen auf der Oberfläche des Antikörpers verändert, so dass diese der Oberfläche von Antikörpern des Zielorganismus ähneln. Beim CDR-Grafting werden die erfindungsgemäßen CDR-Regionen in humane variable Regionen eingebracht.

Humanisierte Antikörper, die die erfindungsgemäßen CDR-Regionen enthalten sind ausdrücklich Bestandteile der Erfindung.

Bestandteile der Erfindung sind auch Antikörper, die mit einer Effektorgruppe konjugiert sind. Unter Konjugation ist hierbei die Ankopplung eines Stoffs an einen Antikörper zu verstehen. Die Verbindung des Antikörpers zur Effektorgruppe wird bevorzugt durch Expression als Fusionsprotein oder durch *in vitro* Methoden hergestellt, wobei die Effektorgruppe bevorzugt über Linkergruppen an den Antikörper gebunden wird (beispielsweise über Thioetherbindungen oder Disulfidbindungen). Sie können an den Antikörper auch durch ein intermediäres Trägermolekül, beispielsweise Serumalbumin, gebunden sein. Gegebenenfalls enthält ein Antikörper auch mehrere Effektorgruppen.

Dabei sind die Effektorgruppen bevorzugt pharmazeutisch wirksame Stoffe (Wirkstoffe). Bevorzugte Wirkstoffe umfassen, sind jedoch nicht limitiert auf Toxine, wie Zytostatika, beispielsweise Maytansinoide und Maytansinoid-Analoga, Taxoide, CC-1065 und CC-1065 Analoga, Dolastatin und Dolastatin-Analoga, Methotrexat, Daunorubicin, Doxorubicin, Vincristin, Vinblastin, Melphalan, Mitomycin C, Chlorambucil und Calicheamicin. Die Erfindung umfasst auch Antikörper, welche mit Radionukliden als Effektorgruppen konjugiert sind und deren Verwendung zur Therapie und Diagnostik, insbesondere von Tumoren. Geeignete Radionuklide sind vorzugsweise die radioaktiven Isotope von Technetium, Rhenium, Yttrium, Kupfer, Gallium, Indium, Bismuth und Platin, wie z. B. ^{99m}Tc, ⁹⁰Y, ¹⁸⁶Re, ¹⁸⁸Re, ⁶⁸Ga und ¹¹¹In.

Erfindungsgemäße Effektorgruppen umfassen weiterhin Enzyme (besonders für das ADEPT-System geeignete Enzyme), costimulatorische Moleküle (z.B. CpG) oder auch Nukleinsäuren. Der mit einer Effektorgruppe konjugierte Antikörper kann gegebenenfalls in Form eines Fusionsproteins vorliegen.

Weiterhin Bestandteile der Erfindung sind auch Antikörper, die mit einem weiteren Antikörper oder Antikörperfragment konjugiert sind, welcher bzw. welches gegen ein anderes Antigen als CD33 gerichtet ist. Der Antikörper ist in diesem Fall ein bispezifischer Antikörper. Bevorzugt ist der Antikörper ein single chain bispecific diabody (scBsDb). In diesem Fall sind zwei scFv Fragmente über einen kurzen (bevorzugt 10 bis 20 Aminosäurereste langen) Linker miteinander verbunden. Besonders bevorzugt ist der Antikörper ein single chain bispecific tandem antibody (scBsTaFv). In diesem Fall sind die zwei scFv-Fragmente über eine längere (bevorzugt 18 bis 50 Aminosäurereste lange) Linker verbunden, was in einer besonders flexiblen Struktur resultiert.

Bevorzugt enthalten diese bispezifischen Antikörper neben dem erfindungsgemäßen CD33-Antikörper einen Antikörper oder ein Antikörperfragment, das gegen Oberflächenantigene von Effektorzellen, wie zum Beispiel T Zellen, besonders cytotoxische T Zellen, NK-Zellen, Monozyten, Makrophagen, Dendritische Zellen, oder Granulozyten gerichtet sind. Die Definition von Effektorzellen im erfindungsgemäßen Sinn umfasst alle die Zellen des angeborenen und adaptiven Immunsystems, die Immunreaktionen vermitteln bzw. aktiv daran beteiligt sind. Besonders bevorzugt sind diese Antikörper gegen folgende Oberflächenstrukturen auf Effektorzellen gerichtet: CD3, CD8, CD4, CD25, CD28, CD16, NKG2D, NKp46, NKp44, aktivierende KIR-Rezeptoren (activating Killer Cell Immunoglobulin-like Receptors).

Ebenfalls Bestandteile der Erfindung sind Antikörper, die mit einem Ligand konjugiert sind, der die Aktivität von Effektorzellen durch Bindung an die Oberfläche der Effektorzellen beeinflusst. Der Ligand ist dabei so ausgewählt, dass er spezifisch an Oberflächenstrukturen von Effektorzellen bindet und durch die Bindung Signalkaskaden zur Aktivierung der Effektorzellen auslöst. Bevorzugt ist als Ligand eine Proteinstruktur oder ein Glycan, welcher spezifisch an einen Rezeptor bindet, der spezifisch auf der Oberfläche von Effektorzellen exprimiert wird, wobei der Ligand durch seine Bindung an den Rezeptor eine Aktivierung der Effektorzelle bewirkt. Besonders bevorzugt sind die Proteinstrukturen ausgewählt aus ULB-Ps (z. B. ULB-P2), MICA, MICB, sowie Zytokinen (wie z.B. IL2 und IL 15) und deren Fusionsproteinen.

Die Verbindung des Antikörpers zum weiteren Antikörper, Antikörperfragment oder zur Proteinstruktur wird bevorzugt durch eine Expression als Fusionsprotein oder durch *in vitro* Methoden hergestellt, wobei die weiteren Antikörper, Antikörper oder Proteinstrukturen bevorzugt über Linker, wie Peptidlinker, an den Antikörper gebunden wird.

Die Erfindung umfasst weiterhin Nukleinsäuresequenzen, die für einen erfindungsgemäßen Antikörper kodieren, sowie Vektoren, die die Nukleinsäuresequenzen enthalten.

Der Vektor (Expressionsvektor) ist jeweils bevorzugt ein Plasmid, ein künstliches Chromosom oder auch ein Viruspartikel oder ein anderer Vektor, der eine Expressionskassette enthält, die stabil in das Genom des Wirts (Wirtszelle oder Wirtsorganismus) eingebaut wird.

Bevorzugt enthalten die Nukleinsäuresequenzen, die für einen erfindungsgemäßen Antikörper kodieren folgende Sequenzen, die für die CDR-Regionen der variablen Regionen der schweren Ketten kodieren:
- CDR1 SEQ ID Nr. 17,
- CDR2 SEQ ID Nr. 18,
- CDR3 SEQ ID Nr. 19 und
   folgende Sequenzen, die für die CDR-Regionen der variablen Regionen der leichten Ketten kodieren
- CDR1 SEQ ID Nr. 20,
- CDR2 SEQ ID Nr. 21, und
- CDR3 SEQ ID Nr. 22.

In einer weiteren bevorzugten Ausgestaltung der Erfindung enthalten die Nukleinsäuresequenzen, die für einen erfindungsgemäßen Antikörper kodieren, folgende Sequenz, die für die variable Region der schweren Kette kodiert: SEQ ID Nr. 29 und/oder folgende Sequenz, die für die variable Region der leichten Kette kodiert: SEQ ID Nr. 30.

Bestandteile der Erfindung sind ebenfalls Wirtszellen oder nicht-menschliche Wirtsorganismen, die eine erfindungsgemäße Nukleinsäuresequenz enthalten.

Eine Wirtszelle ist eine natürlich vorkommende Zelle oder eine transformiert oder genetisch veränderte Zelllinie oder ein (multizellulärer) nicht-menschlicher Wirtsorganismus, welche bzw. welcher das erfindungsgemäße Expressionsystem (d. h. mindestens einen Expressionsvektor) enthält. Die Erfindung umfasst dabei transiente Transfektanten (z. B. durch mRNA-Injektion), also Wirte (Wirtszellen oder -organismen), in denen das Expressionsystem als Plasmid oder künstliches Chromosom enthalten ist, sowie Wirte in denen das Expressionsystem stabil in das Genom des Wirtes (oder einzelner Zellen des Wirtes) integriert ist. Die Wirtszelle ist bevorzugt ausgewählt aus Prokaryonten oder Eukaryonten. Bevorzugte Prokaryonten sind ausgewählt aus *Escherichia coli* und *Bacillus subtilis.* Bevorzugte Eukaryonten sind Hefezellen (z. B. *Saccharomyces cerevisiae, Pichia pastoris),* Insektenzellen, amphibische Zellen oder Säugetierzellen, wie z. B. CHO, HeLa, Hek293T, Hek293A. Bevorzugte Wirtsorganismen sind Pflanzen, wie z. B. Mais oder Tabak, Invertebraten oder Vertebraten, insbesondere *Bovidae, Drosophila melanogaster, Caenorhabditis elegans, Xenopus laevis, Medaka,* Zebrafisch oder *Mus musculus,* oder Zellen oder Embryonen der genannten Organismen.

Die Erfindung umfasst weiterhin eine pharmazeutische Zusammensetzung, die einen oder mehrere erfindungsgemäße Antikörper in Assoziation mit einem pharmazeutisch annehmbaren Verdünnungsmittel oder Träger enthält. Bevorzugt liegt die pharmazeutische Zusammensetzung in einer für die intravenöse Verabreichung geeigneten Form vor.

Vorzugsweise umfasst die Zusammensetzung einen chimären oder humanisierten Antikörper mit einer verringerten Immunogenität, welcher die erfindungsgemäßen CDR Regionen enthält.

Die erfindungsgemäßen pharmazeutischen Zusammensetzungen umfassen verschiedene Dosierungsformen. Die pharmazeutischen Zusammensetzungen werden bevorzugt parenteral, besonders bevorzugt intravenös verabreicht. In einer Ausgestaltung der Erfindung liegt die parenterale pharmazeutische Zusammensetzung in einer Darreichungsform vor, die zur Injektion geeignet ist. Besonders bevorzugte Zusammensetzungen sind daher Lösungen, Emulsionen oder Suspensionen des Antikörpers welches in einem pharmazeutisch annehmbaren Verdünnungsmittel oder Träger vorliegt.

Als Träger werden vorzugsweise Wasser, gepuffertes Wasser, 0,4% Salzlösung, 0,3% Glycin und ähnliche Lösungsmittel eingesetzt. Die Lösungen sind steril. Die pharmazeutischen Zusammensetzungen werden durch herkömmliche, gut bekannte Techniken sterilisiert. Die Zusammensetzungen enthalten bevorzugt pharmazeutisch akzeptable Hilfssubstanzen, wie etwa diejenigen, die erforderlich sind um näherungsweise physiologische Bedingungen zu ergeben und/oder die Stabilität des Antikörpers zu erhöhen, wie etwa Mittel zur Einstellung des pH-Werts und Puffermittel, Mittel zur Einstellung der Toxizität und dergleichen, vorzugsweise ausgewählt aus Natriumacetat, Natriumchlorid, Kaliumchlorid, Calciumchlorid und Natriumlactat. Die Konzentrationen der erfindungsgemäßen Antikörper in diesen Formulierungen sind je nach Anwendung variabel, sie betragen vorzugsweise weniger als 0,01 Gewichts-%, bevorzugt mindestens 0,1 Gewichts-%, weiter bevorzugt zwischen 1 und 5 Gewichts-% und sie werden primär auf der Basis von Fluidvolumina, Viskosität, u.ä. ausgewählt oder in Übereinstimmung mit dem jeweiligen Verabreichungsmodus.

Die erfindungsgemäßen Antikörper werden bevorzugt in eine Zusammensetzung aufgenommen, die für eine parenterale Verabreichung geeignet ist. Bevorzugt ist die pharmazeutische Zusammensetzung eine injizierbare gepufferte Lösung, die zwischen 0,1 bis 500 mg/ml Antikörper, besonders bevorzugt zwischen 0,1 bis 250 mg/ml Antikörper, insbesondere zusammen mit 1 bis 500 mmol/l (mM) Puffer enthält. Die injizierbare Lösung kann sowohl in einer flüssigen als auch ein einer lyophilisierten Dosierungsform vorliegen. Der Puffer kann bevorzugt Histidin sein (bevorzugt 1 bis 50 mM. besonders bevorzugt 5 bis 10 mM), bei einem pH-Wert von 5,0 bis 7,0 (besonders bevorzugt bei einem pH-Wert von 6,0).

Andere geeignete Puffer umfassen, sind aber ausdrücklich nicht beschränkt auf, Natriumsuccinat, Natriumcitrat, Natriumphosphat oder Kaliumphosphat. Bevorzugt wird Natriumchlorid zwischen 0 bis 300 mM, besonders bevorzugt 150 mM für eine flüssige Darreichungsform, verwendet. Für eine lyophilisierte Darreichungsform enthält die pharmazeutische Zusammensetzung bevorzugt Kälteschutzmittel, bevorzugt 0 - 10 % Sucrose (besonders bevorzugt 0,5 - 1,0 %). Andere Kälteschutzmittel umfassen Trehalose und Lactose. Für eine lyophilisierte Darreichungsform enthält die pharmazeutische Zusammensetzung bevorzugt Quellmittel, bevorzugt 1 bis 10 % Mannitol. Andere Quellmittel umfassen Glycin und Arginin. Sowohl in flüssigen als auch in lyophilisierten Darreichungsformen werden bevorzugt Stabilisatoren eingesetzt, besonders bevorzugt zwischen 1 bis 50 mM L-Methionin (besonders bevorzugt zwischen 5 und 10mM).

In einer bevorzugten Ausführungsform umfasst die pharmazeutische Zusammensetzung den Antikörper in einer Dosismenge von 0,1 mg/kg bis 10 mg/kg pro Anwendung. Besonders bevorzugte Dosismengen umfassen 1 mg/kg Körpergewicht.

Pharmazeutische Zusammensetzungen müssen steril und unter den Herstellungs- und Aufbewahrungsbedingungen stabil sein. Die Zusammensetzung kann formuliert werden als eine Lösung, Mikroemulsion, Dispersion, in Liposomen oder einer anderen geordneten Struktur, die für hohe Konzentrationen an Antikörper geeignet ist. Sterile injizierbare Lösungen können hergestellt werden, indem man den Antikörper in der erforderlichen Menge in einem geeigneten Lösungsmittel aufnimmt, mit einem oder mit einer Kombination der vorstehend aufgezählten Inhaltsstoffe je nach Bedarf, gefolgt von einer Filtersterilisation. Für sterile, lyophilisierte Pulver für die Herstellung von sterilen injizierbaren Lösungen sind die bevorzugten Herstellungsverfahren Vakuumtrocknen und Sprühtrocknen, was ein Pulver des Antikörpers plus etwaigen zusätzlichen gewünschten Inhaltsstoffen aus einer zuvor steril filtrierten Lösung davon ergibt.

Die Erfindung umfasst die erfindungsgemäßen Antikörper zur Verwendung als Arzneimittel.

Die Erfindung umfasst weiter die Verwendung eines erfindungsgemäßen Antikörpers zur Herstellung eines Arzneimittels zur therapeutischen und/oder diagnostischen Anwendung bei Erkrankungen, die mit der Expression von CD33 assoziiert sind, insbesondere bei akuter myeloischer Leukämie (AML).

Die Erkrankungen, die mit der Expression von CD33 assoziiert sind, umfassen Krebserkrankungen, wie akute myeloische Leukämie (AML), chronische myeloische Leukämie (CML) und promyeloische Leukämie (PML) und weitere Erkrankungen, wie das Myelodysplastische Syndrom (MDS). Die Anwendung als Medikament umfasst auch weitere nicht explizit genannte Erkrankungen, die mit der Expression von CD33 assoziiert sind.

Für therapeutische Anwendungen wird eine sterile pharmazeutische Zusammensetzung, enthaltend eine pharmakologisch wirksame Dosismenge eines oder mehrerer erfindungsgemäßer Antikörper einem Patienten verabreicht, um vorstehend beschriebene Erkrankungen zu behandeln.

Erfindungsgemäß wird besonders eine Verwendung der Antikörper bevorzugt, die zu einem Targeting von CD33-exprimierenden Zellen führt. Dafür wird durch die Verabreichung des Antikörpers eine Bindung über CD33 an die Zielzellen erreicht und durch den Fc-Teil oder durch die Effektorgruppen an den Antikörpern eine Abtötung der Zielzelle erzielt. Dies erfolgt entweder durch die Rekrutierung von Effektorzellen oder durch den gezielten Transport von pharmakologischen Wirkstoffen (z.B. Toxine) an die CD33-positive Zelle und die dortige Freisetzung.

In einer besonderen Ausgestaltung der Erfindung wird ein Antikörper in Form eines bispezifischen Antikörpers zur Behandlung von CD33-assoziierten Erkrankungen verwendet. Dabei enthält der bispezifische Antikörper in einer besonders bevorzugten Ausgestaltung der Erfindung ein erfindungsgemäßes scFv-Fragment und ein scFv-Fragment, welches gegen eine Oberflächenstruktur auf NK-Zellen gerichtet ist, bevorzugt gegen ULB-P2. Bevorzugt wird der bispezifische Antikörper zur Behandlung von AML verwendet.

In einer weiteren besonders bevorzugten Ausgestaltung der Erfindung enthält der bispezifische Antikörper ein erfindungsgemäßes scFv-Fragment und ein scFv-Fragment, welches gegen eine Oberflächenstruktur auf T-Zellen gerichtet ist, bevorzugt gegen CD3 oder CD8. Bevorzugt wird der bispezifische Antikörper zur Behandlung von AML verwendet.

Neben dem Einsatz in der Medizin für therapeutische Zwecke eignen sich die erfindungsgemäßen Antikörper für die Diagnostik, die biologische Forschung und andere Anwendungen in denen der Nachweis von CD33 interessant ist. Derartige Anwendung sind insbesondere Westernblot, Immunfärbungen von Zellen (z. B. für die Durchflusszytometrie und Mikroskopie) und ELISA.

Zur Erfindung gehört auch ein Verfahren zur Herstellung eines erfindungsgemäßen Antikörpers, bei dem man eine Wirtszelle oder einen nicht-menschlichen Wirtsorganismus Bedingungen aussetzt, die der Expression und gegebenenfalls Sekretion des Antikörpers zuträglich sind und gegebenenfalls den Antikörper wenigstens teilweise aufreinigt. Bevorzugt werden dafür werden Wirtszellen in einem Selektivmedium gezüchtet, das auf das Wachstum der Zellen selektiert, die einen Expressionsvektor enthalten. Die Expression der Gensequenzen des Expressionsvektors resultiert in der Produktion der Antikörper oder Fusionsproteine. Die exprimierten Antikörper oder Fusionsproteine werden dann durch ein beliebiges konventionelles Verfahren isoliert und gereinigt, einschließlich Extraktion, Präzipitation, Chromatographie, Elektrophorese oder auch durch Affinitätschromatographie.

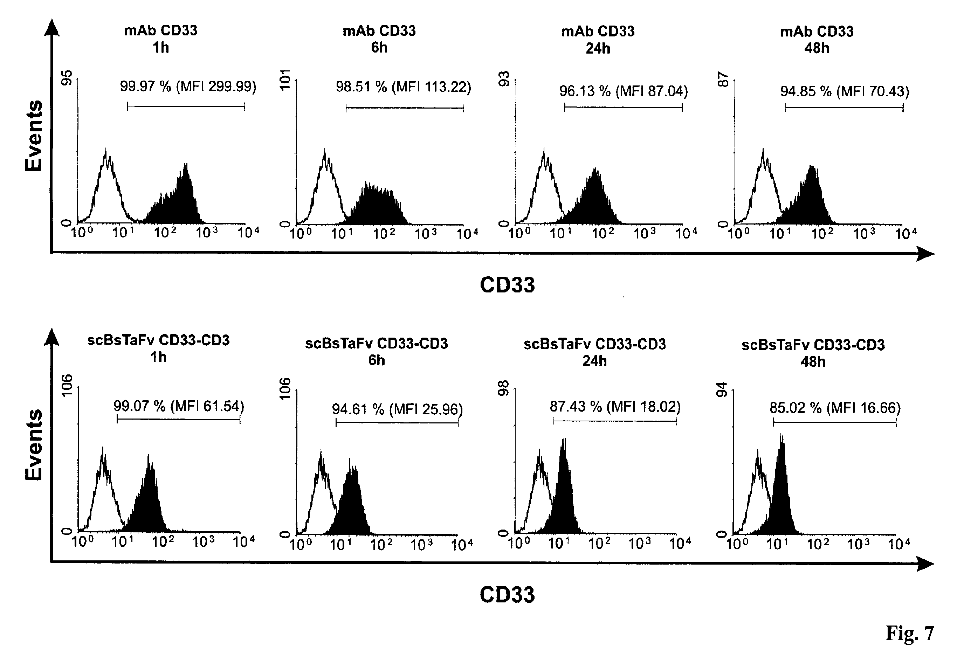

Die erfindungsgemäßen Antikörper werden nur langsam von CD33-positiven Zellen endozytiert (Fig. 7). Dies hat den Vorteil, dass die erfindungsgemäßen Antikörper bzw. Antikörperfragmente bei einer therapeutischen Anwendung über einen längeren Zeitraum an der Oberfläche der CD33-positiven Zellen verbleiben. Dadurch sind sie lange für Effektorzellen zugängig, die ihrerseits wiederum die Immunreaktion gegen die CD33-positiven Zellen (Zielzellen) vermitteln. Dies erhöht die Effektivität einer Therapie. Antikörper, die schnell endozytiert werden, sind für eine solche Anwendung ungeeignet. Insofern heben sich die erfindungsgemäßen Antikörper durch diese besonderen Eigenschaften von den aus dem Stand der Technik vorbekannten Antikörpern ab. Sie bieten zusätzlich zu ihrer hohen Affinität zu CD33 die Möglichkeit einer effektiven therapeutischen Anwendung, deren Wirkmechanismus auf der Rekrutierung von Effektorzellen beruht.

Anhand folgender Figuren und Ausführungsbeispiele soll die Erfindung näher erläutert werden, ohne die Erfindung auf diese zu beschränken:

### Figuren

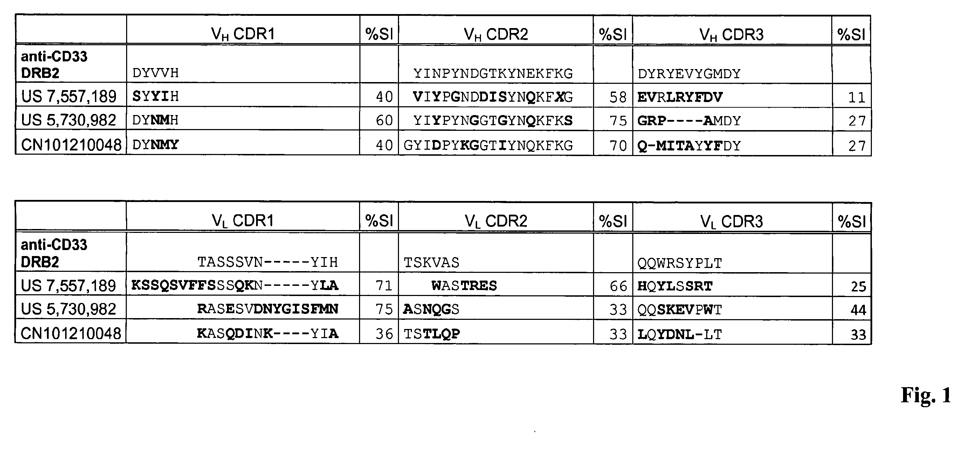

- Figur 1:: Figur 1 zeigt einen Sequenzvergleich der CDR-Regionen des erfindungsgemäßen Antikörpers anti-CD33DRB2, mit den CDR-Regionen der variablen Regionen der leichten und schweren Ketten von aus dem Stand der Technik bekannten anti-CD33 Antikörpern. Dargestellt ist ebenfalls die prozentuale Sequenzidentität (%SI) der Aminosäuresequenzen zu den erfindungsgemäßen Sequenzen.

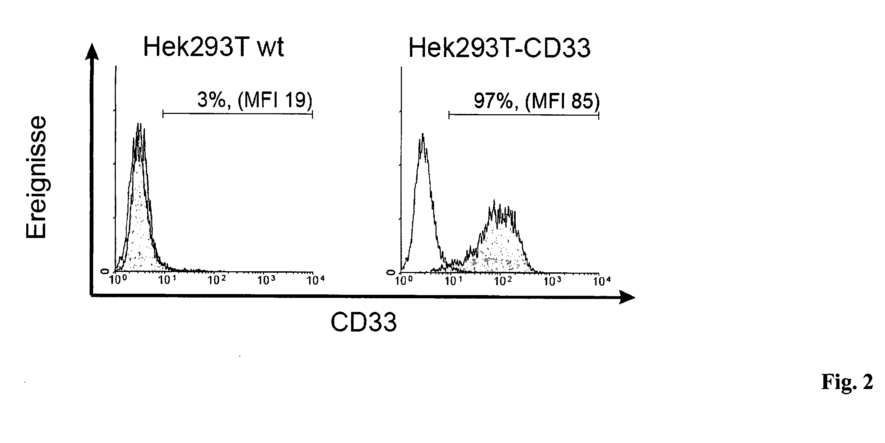

- Figur 2:: Figur 2 zeigt die Bindung des anti-CD33DRB Antikörpers aus dem Überstand der Hybridome an CD33⁺-Hek293T Zellen. Dabei zeigt Figur 2 die Bindung eines Antikörpers mit den CDR-Regionen des anti-CD33DRB2. Dargestellt ist die mittlere Fluoreszenzintensität (MFI) der durchflusszytometrischen Analyse, wobei links die Bindung an Wildtyp Hek293T Zellen (wt) und rechts die Bindung an CD33⁺-Hek293T Zellen (Hek293T-CD33) aufgezeigt ist.
- Figur 3:: Figur 3 zeigt die Bindung von anti-CD33 Antikörpern an HL60 Zellen. Dabei zeigt (A) die Bindung eines kommerziellen anti-CD33-Antikörpers und (B) die Bindung des Antikörpers aus mit den CDR-Regionen von anti-CD33DRB2. Dargestellt ist die mittlere Fluoreszenzintensität (MFI) der durchflusszytometrischen Analyse.

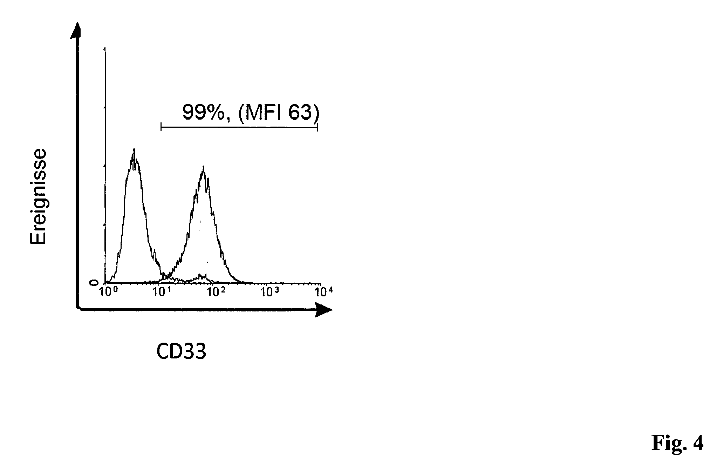

- Figur 4:: Figur 4 zeigt den Vergleich der Bindung eines scFv-Fragments mit den CDR-Regionen des anti-CD33DRB2 an CD33⁺-Hek293T Zellen. Dargestellt ist die mittlere Fluoreszenzintensität (MFI) der durchflusszytometrischen Analyse.

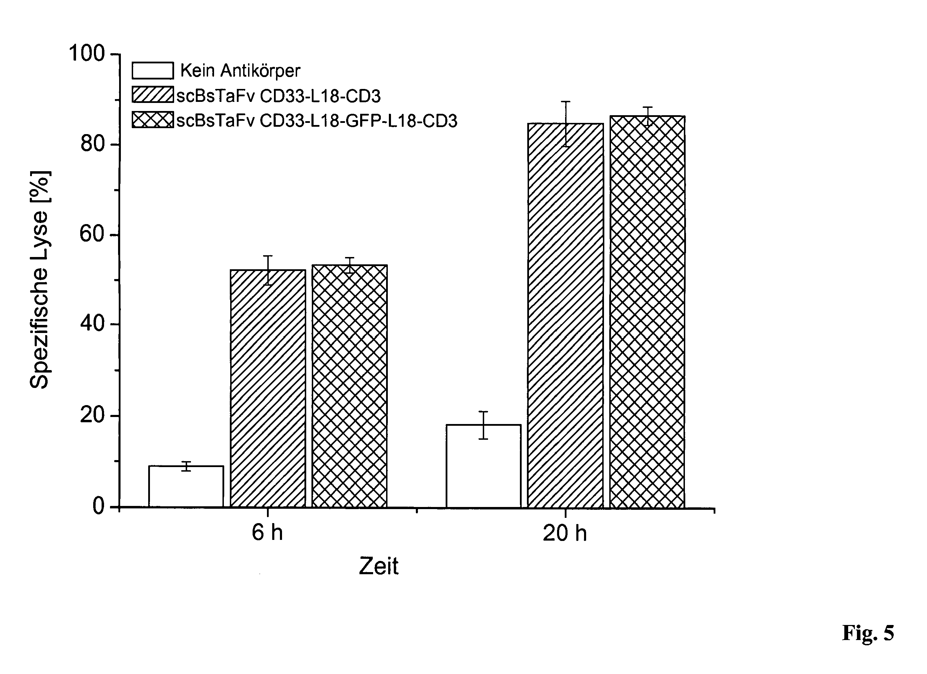

- Figur 5:: Figur 5 zeigt die T-Zell-vermittelte spezifische Lyse von CD33⁺-Hek293T Zellen, welche durch zwei verschiedene scBsTaFv, die gegen CD33 (mit den CDR-Regionen des anti-CD33DRB2) und CD3 gerichtet sind, gesteuert wird. Dargestellt ist der Anteil der CD33⁺-Hek293T-Zellen, die von der T-Zell-vermittelten spezifischen Lyse betroffen sind.

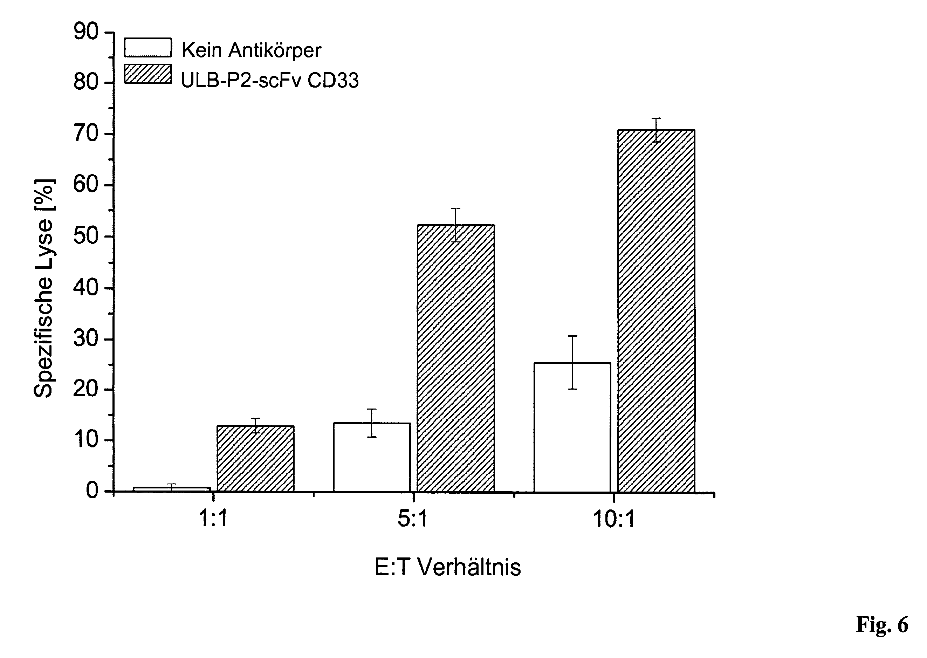

- Figur 6:: Figur 6 zeigt die NK-Zell-vermittelte spezifische Lyse von CD33⁺-P815 Zellen, welche durch ein bispezifisches rekombinantes Protein aus ULB-P2 und scFv CD33 (mit den CDR-Regionen des anti-CD33DRB2 gesteuert wird. Dargestellt ist der Anteil der CD33⁺-Hek293T-Zellen, die von der NK-Zell-vermittelten spezifischen Lyse betroffen sind. Die Experimente wurden in unterschiedlichen Verhältnissen zwischen Effektorzellen (NK Zellen) und Zielzellen (CD33+-P815 Zellen) durchgeführt (E:T Verhältnis, Effektorzellen zu Zielzellen-Verhältnis).
- Figur 7:: Figur 7 zeigt die Verweildauer von erfindungsgemäßen anti-CD33 Antikörpern auf der Oberfläche von CD33-positiven Blasten eines Leukämiepatienten mit AML. Oben: Bindung von anti-CD33DRB2 (aus Beispiel 1) jeweils 1, 6, 24 und 48 h nach Inkubation mit CD33-positiven Blasten (schwarz). Der Nachweis erfolgte durch Färbung mit PE-markiertem antimurinem IgG Antikörper (weiß: Negativkontrolle ohne anti-CD33 Antikörper). Unten: Bindung von CD3xCD33 scBsTaFv (aus Beispiel 4) jeweils 1, 6, 24 und 48 h nach Inkubation mit CD33-positiven Blasten (schwarz). Der Nachweis erfolgte durch Färbung mit PE-markiertem anti-myc Antikörper (weiß: Negativkontrolle ohne CD3xCD33 scBsTaFv).

### Beispiel 1: Herstellung und Charakterisierung monoklonaler muriner anti-CD33DRB Antikörper

Zur Herstellung von neuen anti-CD33 Antikörpern wurde die murine Fibroblasten-Zellinie A9 (ATCC CCL-1.4, American Type Culture Collection, Rockville, Md., USA) stabil mit humanem CD33 transduziert. Mit den so erhaltenen Zellen wurden drei Mäuse immunisiert. Nach der Immunisierung wurden Seren der immunisierten Mäuse auf mit Hek293T-Zellen (ATCC CRL-11268), die ebenfalls mit humanem CD33 transduziert wurden (CD33⁺-Hek293T Zellen), in verschiedenen Verdünnungen aufgebracht und eine Immunfluoreszensfärbung durchgeführt. Das Serum der Maus #3 zeigte bei der höchsten Verdünnung von 1:1000 signifikant die höchste Reaktivität, so dass die Splenozyten dieser Maus für die Hybridomafusion eingesetzt wurden. Dazu wurde die murine Myelomzelllinie X63Ag8.653 (ATCC CRL 1580) in der logarithmischen Wachstumsphase mit Splenozyten der Maus #3 fusioniert und anschließend in einem Selektionsmedium (HAT Medium) kultiviert. Nach zweiwöchiger Kultivierung wurde die Antikörperproduktion der Hybridome getestet. Es wurde ein Hybridom identifiziert, welches sich durch eine besonders hohe Affinität zu CD33 auszeichne (anti-CD33DRB2).

Die Sequenzierung der variablen Regionen der leichten und schweren Ketten zeigt folgende Sequenzen:
- anti-CD33DRB2: V_{H} eine Sequenz gemäß SEQ ID Nr. 29, V_{L} eine Sequenz gemäß SEQ ID Nr. 30. Die CDR-Regionen der variablen Regionen der leichten und schweren Ketten weisen folgende Gensequenzen auf:
- anti-CD33DRB2: V_{H}: CDR1 eine Sequenz gemäß SEQ ID Nr. 17, CDR2 eine Sequenz gemäß SEQ ID Nr. 18, CDR3 eine Sequenz gemäß SEQ ID Nr. 19; V_{L}: CDR1 eine Sequenz gemäß SEQ ID Nr. 20, CDR2 eine Sequenz gemäß SEQ ID Nr. 21, CDR3 eine Sequenz gemäß SEQ ID Nr. 22.

Diese kodieren für folgende Aminosäuresequenzen, die die spezifischen CDR-Regionen enthalten:
- anti-CD33DRB2: V_{H} eine Sequenz gemäß SEQ ID Nr. 13, V_{L} eine Sequenz gemäß SEQ ID Nr. 14
- und die CDR-Regionen von
- anti-CD33DRB2: V_{H}: CDR1 eine Sequenz gemäß SEQ ID Nr. 1, CDR2 eine Sequenz gemäß SEQ ID Nr. 2, CDR3 eine Sequenz gemäß SEQ ID Nr. 3; V_{L}: CDR1 eine Sequenz gemäß SEQ ID Nr. 4, CDR2 eine Sequenz gemäß SEQ ID Nr. 5, CDR3 eine Sequenz gemäß SEQ ID Nr. 6.

Die Aminosäuresequenzen der CDR-Regionen des erfindungsgemäßen Antikörpers unterscheiden sich deutlich von bisher bekannten anti-CD33-Antikörpern (Figur 1).

Die Bindung der Antikörper aus den Hybridomüberständen an CD33⁺-Hek293T-Zellen und an die CD33+ humane Zelllinie HL60 wurde in FACS-Analysen nachgewiesen (Figuren 2 und 3).

Zur Bereitstellung monoklonaler Antikörper aus den Hybridomzellen wurden diese rekloniert.

### Beispiel 2: Herstellung eines scFv-Konstrukts von anti-CD33DRB2

Die kodierenden Bereiche der schweren und leichten Ketten der variablen Regionen des Antikörpers anti-CD33DRB2 aus Beispiel 1 wurden mit spezifischen Primern amplifiziert und zur Expression in eukaryotischen Zellen in einen pSecTag2B Vektor kloniert. Im die Bindungsstärke der scFv (single chain variable fragments) zu überprüfen, wurden diese in Hek293T Zellen exprimiert. Dafür wurde der Vektor (pSecTag2B enthaltend die kodierenden Bereiche) in *E. coli Top10F* Bakterien transformiert und die Plasmide präpariert. Die erhaltenen Expressionsvektoren, die für die scFv Antikörper kodieren, wurden durch Transfektion mit Polyethylenimin (PEI) in Hek293T Zellen eingebracht. Nach fünftägiger Kultur der transfizierten Zellen wurde der Überstand abgenommen und die Antikörper aufgereinigt.

Die erhaltenen scFv Antikörper, im folgenden anti-CD33DRB2 scFv genannt, wurden in einer Immunfluoreszenzfärbung auf ihre Bindung an CD33⁺-Hek293T Zellen getestet und mit den monoklonalen anti-CD33DRB2 verglichen (Figur 4). Der monoklonale Antikörper als auch der scFv Antikörper wiesen eine vergleichbare Bindung an CD33+-Hek293T Zellen auf, wobei die Anzahl der gebunden Zellen gleich war, die Bindungsstärke, die sich in der mittleren Fluoreszenzintensität äußert, beim scFv Antikörper leicht reduziert war. Dies erklärt sich dadurch, dass der scFv Antikörper nur mit einem Paar aus variabler Region der leichten und schweren Kette an das Antigen bindet.

### Beispiel 3: Herstellung verschiedener bispezifischer CD3xCD33 Antikörper und Immunotargeting von T Zellen

Zum Einsatz als Targetingkonstrukt zum Targeting von CD33⁺ Zellen wurde ein bispezifischer Antikörper (single chain bispecific diabody, scBsDb), welche mit einem Arm an CD33 und mit dem anderen Arm an CD3 binden, hergestellt. Die Domäne, die an CD33 bindet, enthält die variable Region des anti-CD33DRB2 aus Beispiel 1 und 2. Sie dient zur Bindung an die Zielzellen, beispielsweise Tumorzellen. Die andere Domäne bindet an CD3, einen Bestandteil des T Zell Rezeptor Komplexes, und dient zur Aktivierung von T Zellen. Dadurch wird die Rekrutierung von T Zellen an die Zielzellen ermöglicht und erlaubt dadurch die Bekämpfung der Zielzellen durch die T Zellen.

Zur Herstellung des Antikörpers wurde eine äußere Kassette für den ersten Antikörper, enthaltend die variable Region der schweren Kette (V_{H}), welche proximal von der variablen Region der leichten Kette (V_{L}) positioniert war, wobei V_{H} und V_{L} über einen Glycin-Serin-Linker aus (Gly₄Ser)₃, verbunden sind. Die innere Kassette enthielt die Reihenfolge der variablen Regionen der leichten und schweren Kette des zweiten Antikörpers in reversierter Form und einen Glycin-Serin-Linker aus (Gly₄Ser)₅, um eine korrekte Faltung der scBsDb zu gewährleisten. So wurden zwei unterschiedliche bispezifische Antikörper hergestellt, die sich in der Anordnung der variablen Regionen der anti-CD33 und anti-CD3 Antikörper unterscheiden:
CD33xCD3 scBsDb (äußere Kassette CD33 - innere Kassette CD3):
   V_{H}CD33-(Gly₄Ser)_V_{L}CD3_{_}(Gly₄Ser)₅- V_{H}CD3-(Gly₄Ser)_V_{L}CD33
CD33xCD3 scBsDb (äußere Kassette CD3 - innere Kassette CD33):
   V_{H}CD3-(Gly₄Ser)_V_{L}CD33_(Gly₄Ser)₅- V_{H}CD33-(Gly₄Ser)_V_{L}CD3.

Die kodierenden Sequenzen der CD33xCD3 scBsDb und CD3xCD33 scBsDb wurden in einen retroviralen pcz-CFG 5.1 Vektor kloniert. Die beiden Konstrukte wurden in eine packaging Zelllinie eingebracht und die erhaltenen viralen Partikel wurden in Hek293T Zellen eingebracht, wo deren kodierende Sequenz stabil in das Genom eingebracht wurde und somit stabile Zellinien erhalten wurden, die die beiden Antikörper, CD33xCD3 scBsDb und CD3xCD33 scBsDb, exprimieren. Diese wurden über eine Ni-NTA-Säule aufgereinigt.

Die so erhaltenen bispezifischen Antikörper enthielten außer der vollständigen Form ein weiteres 37 kDa großes Produkt, welches vermutlich durch Proteolyse entstand und die Zytotoxizität erheblich negativ beeinflusste. Die Zytotoxizität wurde durch einen ⁵¹Cr-Freisetzungs-Assay mit ⁵¹Cr-CD33⁺-Hek293T Zellen bestimmt. CD33xCD3 scBsDb und CD3xCD33 scBsDb konnten in *in vitro* Cokulturen von CD33⁺-Hek293T Zellen und PBMC eine spezifische T-Zell vermittelte Lyse der CD33⁺-Hek293T Zellen von 27 % bzw. 23 % erreichen.

### Beispiel 4: Herstellung von CD3xCD33 Tandemantikörper (scBsTaFv) und Immunotargeting von T Zellen

Um die T-Zell vermittelte Zytotoxizität weiter zu erhöhen wurden Tandemantikörper mit größeren Linkerstrukturen konstruiert, um die Proteolyse zu vermeiden. Bei den bispezifischen Tandemantikörpern (scBsTaFv) sind die variablen Regionen des anti-CD33 Antikörpers und des anti-CD3 Antikörpers auf einer Polypeptidkette angeordnet, wobei die beiden scFv Fragmente mit einem 18 Aminosäuren langen Linker L18 zur korrekten Faltung verknüpft sind. Ein weiteres Konstrukt enthielt außer L18 noch das grün fluoreszierende Protein (GFP). Beide Konstrukte, CD33-L18-CD3 und CD33-L18-GFP-L18-CD3, wurden analog zu Beispiel 3 exprimiert und aufgereinigt. Die Zytotoxiziät wurde ebenfalls analog zu Beispiel 3 mit einem ⁵¹Cr-Freisetzungs-Assay bestimmt (**Figur** 5). Die spezifische Lyse der CD33+-Hek293T Zellen beträgt bei diesen Antikörperkonstrukten bis zu 86% bei 20stündiger *in vitro* Kultur.

### Beispiel 5: Herstellung von ULB-P2xCD33 Tandemantikörpern (scBsTaFv) und Immunotargeting von NK Zellen

Um ein Immunotargeting von NK-Zellen zu erreichen wurden Tandemantikörper konstruiert, die CD33 und ULB-P2 enthalten. ULB-P2 ist der Ligand des aktivierenden NK Zell Rezeptors NKG2D. Durch die Kombination von einem aktivierenden Liganden und einem anti-CD33 scFv Antikörper in einem Tandemantikörper wird ein Immunotargeting von NK Zellen an CD33⁺ Zielzellen vermittelt. Das Konstrukt des Tandemantikörpers enthält ULB-P2, einen kurzen (Gly₄Ser) Linker, und die V_{H} und V_{L} Region des anti-CD33DRB2, welche über eine lange Linkerstruktur (Gly₄Ser)₃ verknüpft sind, sowie ein Myc-Tag und sechs His-Tags zur Aufreinigung. Die Antikörper wurden analog zu den Beispielen 3 und 4 in einen Expressionsvektor eingebracht, exprimiert und aufgereinigt.

Die NK-Zell vermittelte Zytotoxizität wurde mit einem ⁵¹Cr-Freisetzungs-Assay in CD33⁺-P815 (ATCC TIB-6) Zellen ermittelt **(****Figur 6****).** In Gegenwart des Tandemantikörpers wurde eine signifikant erhöhte NK-Zell vermittelte Zytotoxizität festgestellt.

### Beispiel 6: Erfindungsgemäße anti-CD33 Antikörper werden nur schwach von Krebszellen endozytiert

Es ist wünschenswert, dass bei einer therapeutischen Behandlung von CD33-assoziierten Erkrankungen, z.B. akuter myeloischer Leukämie (AML), die Verweilzeit der zur Therapie eingesetzten Antikörper an der Zelloberfläche hoch ist. Dies bewirkt, dass Effektorzellen effektiv an die Krebszellen rekrutiert werden können. Bei einer rapiden Endozytose der Antikörper durch die Krebszellen wären die Bindungsstellen für die Effektorzellen nach kurzer Zeit nicht mehr zugängig und das Targeting der Effektorzellen wäre folglich weniger effektiv.

Um zu demonstrieren, dass erfindungsgemäße Antikörper nur mit geringer Geschwindigkeit von CD33-positiven Krebszellen endozytiert werden, wurden CD33-positive Blasten von Leukämiepatienten mit einen monoklonalen anti-CD33DRB2 Antikörper (wie in Ausführungsbeispiel 1 beschrieben) oder mit einem CD3xCD33 scBsTaFv Tandemantikörper (wie in Ausführungsbeispiel 4 beschrieben) bei 4°C für 1 h inkubiert. Per FACS-Analyse wurde die Präsenz der anti-CD33 Antikörper auf der Oberfläche der CD33-positiven Blasten detektiert, indem eine Färbung der anti-CD33 Antikörper mit einem PE-markierten anti-Maus IgG Antikörper (für den monoklonalen anti-CD33DRB2, Fig. 7 oben, schwarzes Histogramm) bzw. mit einem PE-markierten anti-myc Antikörper (für den Tandemantikörper, Fig. 7 unten, schwarzes Histogramm) durchgeführt wurde. Parallel dazu wurden CD33-positive Blasten desselben Patienten als Negativkontrolle ohne Inkubation mit anti-CD33 Antikörpern mit jeweils denselben Antikörpern gefärbt (Fig. 7, jeweils weißes Histogramm).

Die Antikörper konnten selbst 48h nach der Kontaktierung mit den CD33-positiven Blasten auf der Oberfläche nachgewiesen werden. Die Antikörper - sowohl der monoklonale Antikörper als auch das bispezifische Antikörperderivat - wurden innerhalb dieser Zeit zum überwiegenden Teil nicht von den CD33-positiven Blasten endozytiert. Es ist also für 48h sichergestellt, dass sich die Antikörper für 48h auf der Oberfläche der abzutötenden Zellen befinden und daher über einen langen Zeitraum in der Lage sind, für eine Rekrutierung und Bindung von Effektorzellen zu dienen (bei den monoklonalen Antikörpern z.B. über Fc-Rezeptoren; bei den bispezifischen Antikörperderivaten durch spezifische Bindung der weiteren Antikörper).

## Patentansprüche

1. Anti-CD33-Antikörper enthaltend komplementaritätsbestimmende Regionen (complementarity determining regions, CDRs), **dadurch gekennzeichnet, dass** die CDRs die folgenden Sequenzen umfassen:
a) variable Region der schweren Kette (V_{H})
CDR1 DYWH,
CDR2 YINPYNDGTKYNEKFKG,
CDR3 DYRYEVYGMDY, und
b) variable Region der leichten Kette (V_{L})
CDR1 TASSSVNYIH,
CDR2 TSKVAS,
CDR3 QQWRSYPLT.

2. Antikörper nach Anspruch 1, enthaltend die folgende, gegebenenfalls humanisierte, Struktur:
- eine variable Region der schweren Kette mit der Sequenz gemäß SEQ ID No 13
- und eine variable Region der leichten Kette mit der Sequenz gemäß SEQ ID No 14.

3. Antikörper nach Anspruch 1 oder 2, enthaltend zusätzlich mindestens einer der folgenden Strukturen
- eine konstante Region einer schweren Kette eines humanen IgG,
- eine Region C_{L} der humanen leichten Kappa-Kette und/oder
- eine humane IgG3 Hinge-Region,
gegebenenfalls in Form eines F(ab')₂-Fragments.

4. Antikörper nach einem der Ansprüche 1 bis 3 in Form eines scFv-Fragments oder in Form eines F(ab')₂-Fragments.

5. Antikörper nach einem Ansprüche 1 bis 4, **dadurch gekennzeichnet, dass** er mit einer Effektorgruppe oder einem weiteren Antikörper oder Antikörperfragment konjugiert ist, welcher gegen ein anderes Antigen als CD33 gerichtet ist.

6. Antikörper nach Anspruch 5, wobei die Effektorgruppe ausgewählt ist aus Toxinen, Enzymen, co-stimulierenden Molekülen, Radionukliden und Nukleinsäuren, gegebenenfalls in Form eines Fusionsproteins.

7. Antikörper nach einem der Ansprüche 1 bis 6, **dadurch gekennzeichnet, dass** er mit einem Ligand konjugiert ist, der spezifisch an Effektorzellen bindet und dadurch deren Aktivität beeinflusst.

8. Nukleinsäuresequenz codierend für einen Antikörper nach Anspruch 1 bis 7.

9. Vektor enthaltend eine Nukleinsäuresequenz nach Anspruch 8.

10. Wirtszelle oder nicht-menschlicher Wirtsorganismus enthaltend eine Nukleinsäuresequenz nach Anspruch 8 oder einen Vektor nach Anspruch 9.

11. Pharmazeutische Zusammensetzung, enthaltend einen Antikörper nach einem der Ansprüche 1 bis 7 in Assoziation mit einem pharmazeutisch annehmbaren Verdünnungsmittel oder Träger, gegebenenfalls in einer für die intravenöse Verabreichung geeigneten Form.

12. Antikörper nach einem der Ansprüche 1 bis 7 zur Verwendung als Arzneimittel.

13. Verfahren zur Herstellung eines Antikörpers nach einem Ansprüche 1 bis 7, bei dem man:
a. eine Wirtszelle oder einen nicht-menschlichen Wirtsorganismus nach Anspruch 10 Bedingungen aussetzt unter denen eine Expression und gegebenenfalls eine Sekretion des Antikörpers stattfindet und gegebenenfalls
b. den Antikörper wenigstens teilweise aufreinigt.

## Claims

1. Anti-CD33 antibody containing regions determined by complementarity (complementary determining regions, CDRs), **characterized in that** the CDRs comprise the following sequences:
a) variable region of the heavy chain (V_{H})
CDR1 DYVVH,
CDR2 YINPYNDGTKYNEKFKG,
CDR3 DYRYEVYGMDY,and
b) variable region of the light chain (V_{L}) :
CDR1 TASSSVNYIH
CDR2 TSKVAS,
CDR3 QQWRSYPLT.

2. Antibody according to claim 1, containing the following, optionally humanized, structure:
- a variable region of the heavy chain with the sequence according to SEQ ID NO. 13
- and a variable region of the light chain with a sequence according to SEQ ID NO. 14.

3. Antibody according to claim 1 or 2, containing additionally at least one of the following structures
- a constant region of a heavy chain of a human IgG,
- a region C_{L} of the human kappa light chain
and/or
- a human IgG3 hinge region,
optionally in the form of a F(ab')₂ fragment.

4. Antibody according to one of the claims 1 to 3 in the form of an scFv fragment or in the form of a F(ab')₂ fragment.

5. Antibody according to one of the claims 1 to 4, **characterized in that** it is conjugated with an effector group or a further antibody or antigen binding fragment that is specific to another antigen than CD33.

6. Antibody according to claim 5, wherein the effector group is selected from toxins, enzymes, co-stimulating molecules, radionuclides and nucleic acids, optionally in the form of a fusion protein.

7. Antibody according to one of the claims 1 to 6, **characterized in that** it is conjugated with a ligand that binds specifically to effector cells and in this way affects their activity.

8. Nucleic acid sequence coding for an antibody according to claim 1 to 7.

9. Vector containing a nucleic acid sequence according to claim 8.

10. Host cell or non-human host organism containing a nucleic acid sequence according to claim 8 or a vector according to claim 9.

11. Pharmaceutical composition, containing an antibody according to one of the claims 1 to 7 in association with a pharmaceutically acceptable dilution agent or carrier, optionally in a form suitable for intravenous administration.

12. Antibody according to one of the claims 1 to 7 for use as a medicament.

13. A method for preparing an antibody according to claim 1, in which:
a. a host cell or non-human host organism according to claim 10 is exposed to conditions under which an expression and optionally a secretion of the antibody takes place, and optionally
b. the antibody is at least partially purified.

## Revendications

1. Anticorps anti-CD33 contenant des régions de détermination de complémentarité (complementarity determining regions, CDR), **caractérisé en ce que** les CDR comprennent les séquences suivantes :
a) région variable de la chaîne lourde (V_{H})
CDR1 DYVVH,
CDR2 YINPYNDGTKYNEKFKG,
CDR3 DYRYEVYGMDY, et
b) région variable de la chaîne légère (V_{L}) CDR1 TASSSVNYIH,
CDR2 TSKVAS,
CDR3 QQWRSYPLT.

2. Anticorps selon la revendication 1, contenant la structure suivante, éventuellement humanisée :
- une région variable de la chaîne lourde avec la séquence selon SEQ ID N° 13
- et une région variable de la chaîne légère avec la séquence selon SEQ ID N° 14.

3. Anticorps selon la revendication 1 ou 2, contenant en outre au moins une des structures suivantes
- une région constante d'une chaîne lourde d'une IgG humaine,
- une région C_{L} de la chaîne kappa légère humaine et/ou
- une région charnière IgG3 humaine,
éventuellement sous forme d'un fragment F(ab')₂.

4. Anticorps selon l'une des revendications 1 à 3 sous forme d'un fragment scFv ou sous forme d'un fragment F(ab')₂.

5. Anticorps selon l'une des revendications 1 à 4, **caractérisé en ce qu'**il est conjugué à un groupe effecteur ou un autre anticorps ou fragment d'anticorps qui est orienté contre un autre antigène que CD33.

6. Anticorps selon la revendication 5, dans lequel le groupe effecteur est sélectionné parmi des toxines, enzymes, molécules costimulantes, radionucléides et acides nucléiques, éventuellement sous forme d'une protéine de fusion.

7. Anticorps selon l'une des revendications 1 à 6, **caractérisé en ce qu'**il est conjugué à un ligand qui se fixe spécifiquement à des cellules effectrices et influence ainsi leur activité.

8. Séquence d'acide nucléique codant pour un anticorps selon les revendications 1 à 7.

9. Vecteur contenant une séquence d'acide nucléique selon la revendication 8.

10. Cellule souche ou organisme souche non humain contenant une séquence d'acide nucléique selon la revendication 8 ou un vecteur selon la revendication 9.

11. Composition pharmaceutique, contenant un anticorps selon l'une des revendications 1 à 7 en association à un diluant ou support pharmaceutiquement acceptable, éventuellement dans une forme convenant à l'administration par voie intraveineuse.

12. Anticorps selon l'une des revendications 1 à 7 pour l'utilisation en tant que médicament.

13. Procédé de fabrication d'un anticorps selon l'une des revendications 1 à 7, lors duquel on :
a. expose une cellule souche ou un organisme souche non humain selon la revendication 10 à des conditions dans lesquelles une expression et éventuellement une sécrétion de l'anticorps a lieu et éventuellement
b. purifie au moins partiellement l'anticorps.
